# EUROPEAN PATENT SPECIFICATION

(11) **EP 3 212 804 B1**
(45) Date of publication and mention of the grant of the patent: **12.12.2018**
(21) Application number: 15787560.0
(22) Date of filing: 28.10.2015
(51) Int. Cl.: C12Q 1/68

(54) **IMPROVED TREATMENTS OF CANCER RESISTANT TO TAXOIDS**
VERBESSERTE BEHANDLUNGEN VON GEGEN TAXOIDE RESISTENTEM KREBS
TRAITEMENTS AMÉLIORÉS D'UN CANCER RÉSISTANT À DES TAXOÏDES

(30) Priority: 28.10.2014 EP 14306717
(43) Date of publication of application: 06.09.2017
(73) Proprietor: Institut Gustave Roussy, 94800 Villejuif (FR)
(72) Inventor: CHAUCHEREAU, Anne, F-92260 Fontenay-aux-Roses (FR); MARTIN, Nicolas Jean-Paul, F-78130 Les Mureaux (FR)
(74) Representative: Cabinet Becker et Associés
(86) International application number: PCT/EP2015/074926
(87) International publication number: WO 2016/066665

(56) References cited:
- WO-A1-2011/124669
- WO-A1-2014/058317
- WO-A2-2011/152884
- CHEN CHUN-CHIEH ET AL: "Shisa3 Is Associated with Prolonged Survival through Promoting beta-Catenin Degradation in Lung Cancer", AMERICAN JOURNAL OF RESPIRATORY AND CRITICAL CARE MEDICINE, vol. 190, no. 4, August 2014 (2014-08), pages 433-444, XP008176178,
- DATABASE BIOSIS [Online] BIOSCIENCES INFORMATION SERVICE, PHILADELPHIA, PA, US; April 2009 (2009-04), CHEN CHUN-CHIEH ET AL: "A novel tumor suppressor, SHISA3, inhibits the lung cancer invasion and metastasis", XP008176177, Database accession no. PREV200900497141 & PROCEEDINGS OF THE AMERICAN ASSOCIATION FOR CANCER RESEARCH ANNUAL MEETING, vol. 50, April 2009 (2009-04), page 1072, 100TH ANNUAL MEETING OF THE AMERICAN-ASSOCIATION-FOR-CANCER-RESEARCH; DENVER, CA, USA; APRIL 18 -22, 2009 ISSN: 0197-016X
- LEE SOO-CHIN ET AL: "Chemotherapy-induced tumor gene expression changes in human breast cancers", PHARMACOGENETICS AND GENOMICS, LIPPINCOTT WILLIAMS & WILKINS, PHILADELPHIA, PA, US, vol. 19, no. 3, 1 March 2009 (2009-03-01), pages 181-192, XP009150947, ISSN: 1744-6872

## Description

### FIELD OF THE INVENTION

The present invention relates to the field of medicine, in particular of oncology. It concerns improvements in the treatment of cancer with a resistance to molecules of the taxoid family and the selection of patients for such treatments, kits and methods for screening compounds useful to improve treatment with taxoid molecules.

### BACKGROUND OF THE INVENTION

Taxoids (also called taxanes) are diterpenes, namely organic compounds composed of four isoprene units, widely used as cancer chemotherapy agents. The first taxoids agents, paclitaxel (Taxol®) and docetaxel (Taxotere®), were originally produced by the plants of the genus Taxus (yews). Taxoids are considered as the most promising antitumor agents available at hand today (Fauzee NJS et al, Asian Pacific J Cancer Prev, 2011, 12, pp. 837-851). Indeed, despite their side effects, they are one of the most acceptable treatments for metastatic breast, ovarian, prostate and lung carcinomas. The principal mechanism of action of these drugs is the disruption of microtubule function. Microtubules are essential to cell division and taxoids stabilize GDP-bound tubulin in the microtubule, thereby inhibiting the process of cell division. Thus, in essence, taxoids are mitotic inhibitors.

Based on frequency, prostate cancer became the most common non-skin cancer in men in the Western world and the sixth leading cause of cancer-related death among men worldwide (Eeles R. et al, Nat Rev urol., 2014, 11(1), pp. 18-31). Since 2005, more than 60,000 men are touched by prostate cancer each year and 10,000 men died of this disease. The efficiency of docetaxel chemotherapy in prostate cancer has been demonstrated for the first time in 2004 in two clinical trials, i.e. TAX 327 and SWOG 99-16, with an increase in survival. Accordingly, docetaxel became today a treatment of choice of metastatic hormone-refractory prostate cancers and phase III clinical trials (GETUG 12) demonstrated its efficacy for the treatment of high-risk localized prostate cancer. Docetaxel is the standard of care as first-line chemotherapy for progressing patients with metastatic castration-resistant prostate cancer and improves patient survival by months.

More generally, in Europe and the US, docetaxel is currently approved for the treatment of various cancers including prostate cancer, breast cancer, ovarian cancer, lung cancer, gastric cancer and head and neck cancer. However, in spite of the survival benefit provided by this molecule, docetaxel has a great toxicity and almost half of the patients treated with docetaxel develop a resistance to the chemotherapy either from the beginning, or in a secondary way. Docetaxel is indeed not effective on all the types of cancer. For instance, in case of breast cancers, only 30 to 50 % of the metastatic tumours respond to docetaxel. Moreover, docetaxel is often the last line of treatment available. For example, there is today no standard new line of treatment for castration-resistant prostate cancer resistant to docetaxel (Fitzpatrick JM et al, Eur J Cancer Oxf Engl, 2014, 50(9), pp 1617-27).

Clinical drug resistance is a serious obstacle in any cancer chemotherapy and especially when it concerns paclitaxel and docetaxel, two of the most widely used agents for chemotherapy. Consequently, it is extremely important to discover and develop efficient reversal agents so that clinical oncologists can continue treatment of cancer patients with available anticancer drugs, i.e. paclitaxel, docetaxel, cabazitaxel and other taxoids.

Studies have been carried out to determine the role of genes in the docetaxel response. An expression signature of 30 genes was thus determined, based on a genomic analysis with two cell lines (PC3 and DU145) resistant to a docetaxel dose of 11 nM (Patterson et al, Oncogene, 2006, 25, pp. 6113-6122). More recently, the inventors disclosed a signature of 338 genes associated with the docetaxel resistance, based on the comparative micro-array genomic analysis of sensitive and resistant prostate cancer cell lines (international patent application WO 2010/037859). However, the use of these signatures to develop new strategies to overcome drug resistance to taxoids is complex and costly.

Therefore, taxoid resistances are common nowadays and there is an increasing need for improved cancer treatments so as to overcome these resistances and for simple and cheap methods for selecting those patients who will benefit from these improved treatments.

### SUMMARY OF THE INVENTION

The gene LOC152573, also named *SHISA3,* encoding the hypothetical protein BC012029 (hShisa3), was found to be under-expressed in renal cell carcinoma (Hirota et al., Int J Oncol. 2006, 29(4), pp. 799-827) and in prostate cancer cell lines (IGR-CaP1-R, LNCaP-R and PC3-R) resistant to docetaxel (international patent application WO 2010/037859). In lung cancer, hShisa3 was shown to inhibit the Wnt pathway (Chen CC et al, 2014, Am J Respir Crit Care Med, 190, pp. 433-444). *Shisa3* homologues were described in mouse (Furushima et al. Dev Biol. 2007, 306(2), pp. 480-92), chicken (Katoh and Katoh, Int J Mol Med. 2005, 16(1), pp. 181-5) and in *Xenopus* (Yamamoto et al., Cell. 2005, 120(2), pp. 223-35). These genes encode an antagonist against both Wnt and FGF signalings. In *Xenopus,* SHISA is an essential factor for head formation during gastrulation. SHISA antagonizes Wnt and FGF signallings by interacting with immature forms of the Wnt receptor Frizzled and the fibroblast growth factor receptor, eliciting their retention within the endoplasmic reticulum, suppressing their post-transcriptional maturation and their trafficking to the cell surface, and reducing protein expression (Yamamoto et al., Cell. 2005, 120(2), pp. 223-35).

Cancer cells have the property to change their phenotype by engaging themselves in a complex process named Epithelial-to-Mesenchymal transition (EMT) which allows the acquisition of migratory and invasive properties necessary for metastasis dissemination. In the recent years, new evidences indicate that EMT may participate to the development of cancer drug resistances, for example in prostate cancer (Li P et al, Mol Cancer, 2014, 13, p. 55; Feng X et al, Cancer Letter, 2014, 344 (2), pp. 166-173). Some studies have reported that cells which underwent EMT are enriched in markers which delineate a cell population supposed to be responsible for the chemoresistance (Puhr M. et al, Am J Pathol, 2012, 181(6), pp. 2188-201; Marin-Aguilera M et al, Mol Cancer Ther, 2014, 13(5), pp. 1270-84). However, the role of these markers, their relationship with EMT and the regulation of signalling pathways in cancer chemoresistance are still unclear.

The inventors have surprisingly discovered that SHISA3 under-expression in prostate cancer cells (IGR-CaP1 and PC3 models) mediates a TGF-β induced EMT leading to the development of a chemoresistance to docetaxel. The inventors also demonstrate that combined treatment of docetaxel with therapies targeting TGF-β signalling pathway can circumvent docetaxel resistance in prostate cancer, paving a new avenue in the treatment of patients with taxoid resistant cancers. Indeed, SHISA3 under expression can now be used to select taxoid resistant patients who will benefit from a combined treatment of a taxoid family molecule and a compound inhibiting the TGF-β signalling pathway.

The present invention which addresses the needs of the prior art, provides, in a first aspect, an *in vitro* method for selecting a patient affected with a tumor for a treatment with a molecule of the taxoid family in combination with a compound inhibiting the TGF-β signalling pathway or for determining whether a patient affected with a tumor is susceptible to benefit from a treatment with a molecule of the taxoid family in combination with a compound inhibiting the TGF-β signalling pathway, wherein the method comprises:
(a) determining the expression level of SHISA3 in a cancer sample from said patient,
(b) comparing the expression level of SHISA3 to a reference expression level, wherein the under-expression of SHISA3 is predictive that a treatment with a molecule of the taxoid family in combination with a compound inhibiting the TGF-β signalling pathway is indicated for said patient and
(c) selecting patients with under-expression of SHISA3 as suitable for a treatment with a molecule of the taxoid family in combination with a compound inhibiting the TGF-β signalling pathway.

In a second aspect, the invention also provides, a compound inhibiting the TGF-β signalling pathway for use in combination with a molecule of the taxoid family in the treatment of a cancer in which SHISA3 is under-expressed in comparison to a reference expression level.

Preferably, the cancer is resistant to a molecule of the taxoid family.

Preferably, the expression level of SHISA3 is determined by measuring the quantity of SHISA3 protein or SHISA3 mRNA.

Preferably, the reference expression level is the expression level of SHISA3 in cell lines or tumors sensitive to the treatment by the molecule of the taxoid family. More preferably, the cell-line is a cancer cell-line. Even more preferably, the cell line is IGR-CaP1.

Preferably, the molecule of the taxoid family is selected from the group consisting of paclitaxel (taxol®) docetaxel (taxotere®), larotaxel, cabazitaxel (XRP6258), BMS-184476, BMS-188797, BMS-275183, ortataxel, RPR 109881A, RPR 116258, NBT-287, PG-paclitaxel, ABRAXANE®, Tesetaxel, IDN 5390, Taxoprexin, DHA-paclitaxel, and MAC-321, Genexol-PM, LEP (LEP-ETU), Tocosol® paclitaxel, SB-T-1213, SB-T-1214, SB-T-1216, SB-T-1217, SB-T-121303, SB-T-121303021, SB-T-12130301, and a combination thereof. More preferably, the molecule of the taxoid family is docetaxel.

Preferably, the cancer is a solid tumor. More preferably, the cancer is selected from the group consisting of the prostate cancer, the lung cancer, the breast cancer, the gastric cancer, the kidney cancer, the ovarian cancer, the hepatocellular cancer, the osteosarcoma, the melanoma, the hypopharynx cancer, the esophageal cancer, the endometrial cancer, the cervical cancer, the pancreatic cancer, the liver cancer, the brain cancer, the neuroendocrine tumors, the malignant tumor of the muscle, the adrenal cancer, the thyroid cancer, the uterine cancer, the skin cancer, the bladder cancer, and the head and neck cancer. Still more preferably, the cancer is the prostate cancer.

Preferably, the compound inhibiting the TGF-β signaling pathway is an inhibitor of the TGF-β receptor. More preferably the compound inhibiting the TGF-β signaling pathway is an inhibitor of the TGF-β receptor I or II, especially TGF-β receptor I. Even more preferably, the compound inhibiting the TGF-β signaling pathway is an inhibitor of the TGF-β receptor I that can be selected in the group consisting of: LY2157299 (Galunisertib), SB431542, LY2109761, SB525334, SB505124, RepSox, GW788388, LY364947, A 83-01, SD 208, D 4476, TGF-β RI Kinase Inhibitor VIII, and Ki268994. Still preferably, the inhibitor of the TGF-β receptor I is LY2157299.

In a third aspect, the present invention also relates to an *in vitro* method for screening or identifying a compound suitable for improving the treatment of a cancer with a molecule of the taxoid family or for reducing the resistance development during the treatment of a cancer with a molecule of the taxoid family, comprising:
(a) providing a cell-line in which SHISA3 is under-expressed in comparison to a reference expression level, contacting said cell-line with a test compound, determining the activity level of the TGF-β signaling pathway and selecting the compound which inhibits the TGF-β signaling pathway; or
(b) providing a cell-line sensitive to the molecules of the taxoid family, contacting said cell-line with a test compound and a molecule of the taxoid family, determining the expression level of SHISA3 and selecting the compound which inhibits the appearance of an under-expression of SHISA3 or which inhibits the appearance of resistant cells.

In a fourth aspect, the invention relates to a kit for selecting a patient affected with a tumor for a treatment with a molecule of the taxoid family in combination with a compound inhibiting the TGF-β signaling pathway and/or for screening or identifying a compound suitable for improving the treatment of a cancer with a molecule of the taxoid family, wherein the kit comprises detection means selected from the group consisting of a pair of primers, a probe and an antibody specific to SHISA3, and a combination thereof.

In addition, the present invention also relates to the use of the expression level of SHISA3 as a marker for selecting a patient affected with a tumor for a treatment with a molecule of the taxoid family in combination with a compound inhibiting the TGF-β signalling pathway.

Finally, the present invention relates to the use of a kit for selecting a patient affected with a tumor for a treatment with a molecule of the taxoid family in combination with a compound inhibiting the TGF-β signaling pathway and/or for screening or identifying a compound suitable for improving the treatment of a cancer with a molecule of the taxoid family, wherein the kit comprises detection means selected from the group consisting of a pair of primers, a probe and an antibody specific to SHISA3, and a combination thereof.

### BRIEF DESCRIPTION OF THE DRAWINGS

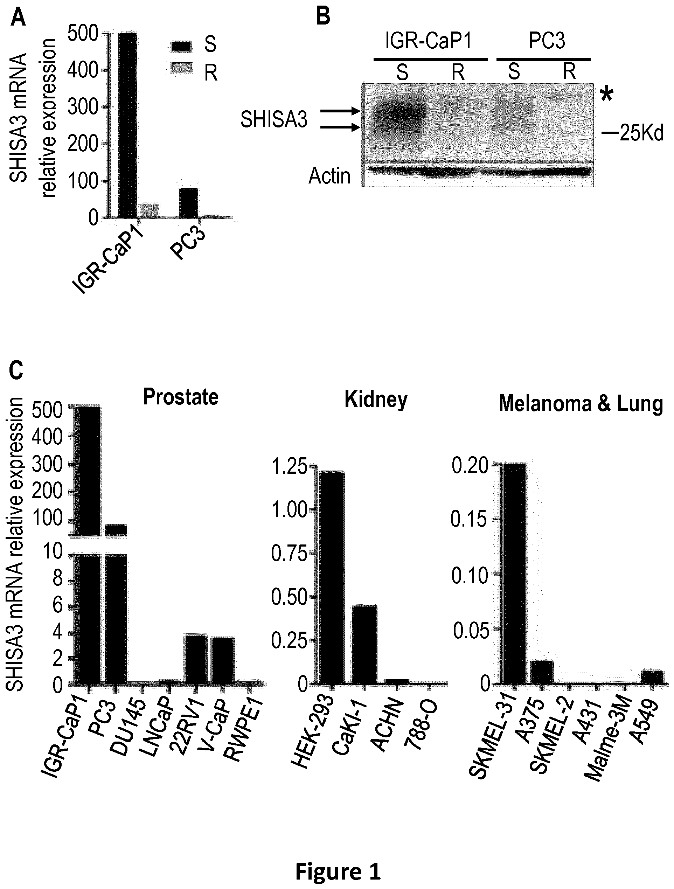

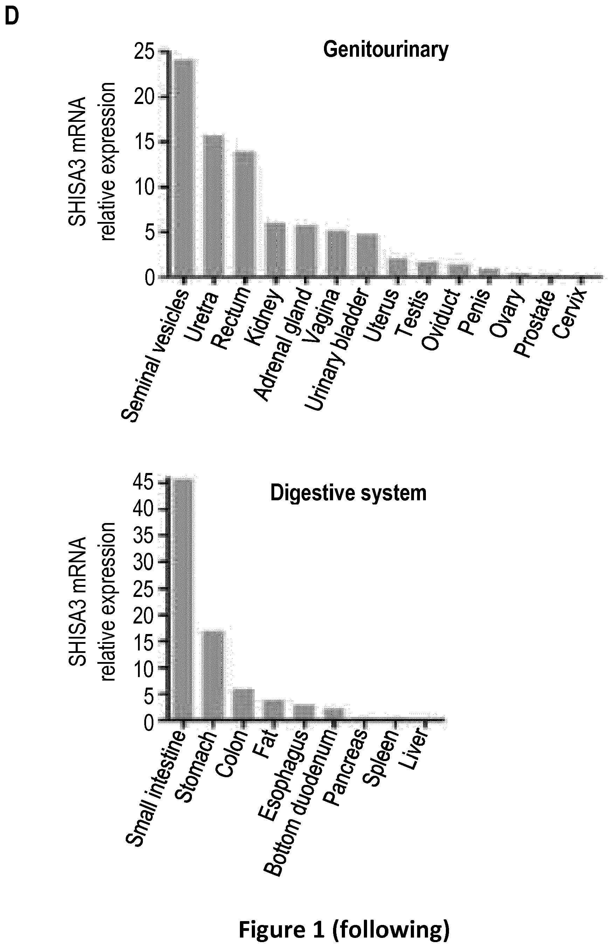

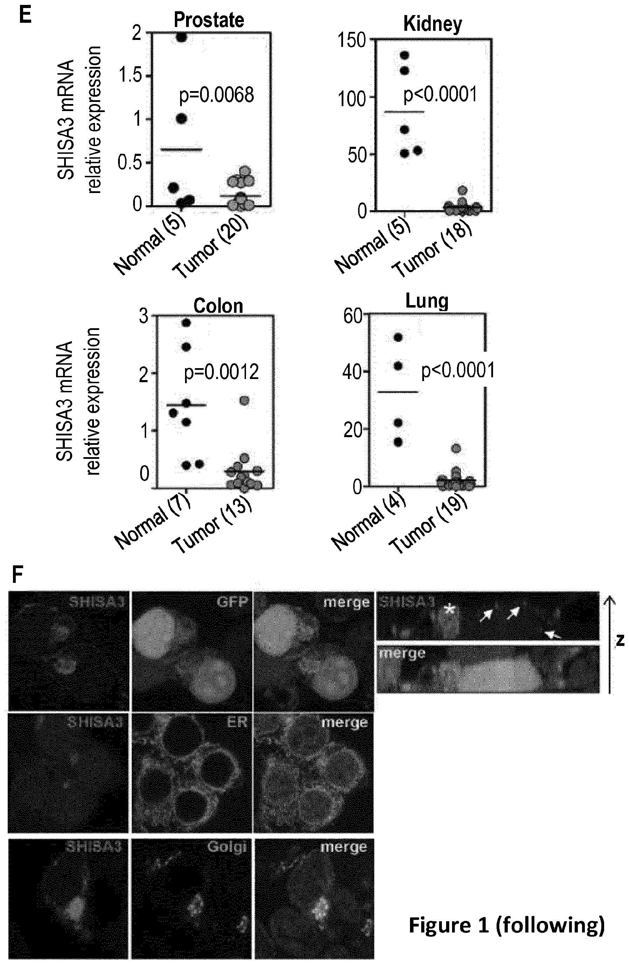

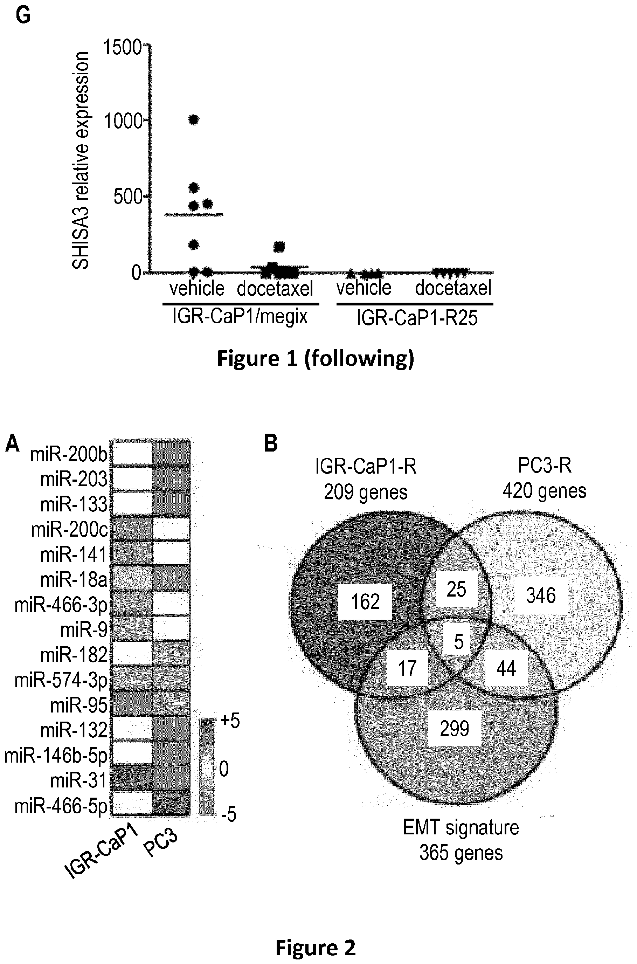

**Figure 1****: Expression of SHISA3 in docetaxel-resistant prostate cancer cells and in other cancers**
   **A.** Real-time qRT-PCR showing the inhibition of *SHISA3* gene expression in docetaxel-resistant IGR-CaP1-R and PC3-R cells. **B.** Whole cell extracts of parental and drug-resistant cells were subjected to immunoblotting with antibodies specific for SHISA3 or β-actin (loading control). Arrows indicate specific bands corresponding to SHISA3, star indicates a non-specific band. **C.** Quantitative RT-PCR shows *SHISA3* expression in several prostate, kidney and melanoma cell lines. **D-E.** *SHISA3* mRNA was assessed by determining ΔCt in normal tissues and in several solid tumors. Results were normalized to the universal RNA included in each panel. In **D,** out of all the tested tissues for *SHISA3* expression, only tissues with the highest expression were shown. In **E,** the bars represent the mean. *P* value was derived from the two-tailed Student's *t* test. **F.** Subcellular localization after transfection of *SHISA3-Flag-GFP* in HEK cells. Cells were analyzed by confocal microscopy. GFP stained the transfected cells, SHISA3 was labeled with anti-SHISA3 antibody (red) and nucleus was stained with Dapi. Z-projections (right) highlighted the two localizations of SHISA3: Arrows show cell membrane speckles and the star shows the intracytoplasmic localization. Co-labeling of SHISA3 with anti-SHISA3 (red) and anti-Calnexin (green), marker of the ER, showed the absence of colocalization of SHISA3 with ER. Co-labeling of SHISA3 (red) and anti-GM130 (green), marker of the Golgi, showed the colocalization of cytoplasmic SHISA3 with Golgi. Secondary antibodies were used as followed: Alexa-568 for SHISA3 and Alexa-647 for Calnexin and GM130. **G.** *SHISA3* mRNA expression was assessed in *vivo* on nude athymic mice models. After injection of docetaxel sensitive (IGR-CaP1) or docetaxel resistant (IGR-CaP1-R25) prostate cancer cell line, xenografted mice received a successive treatment with docetaxel or vehicle when the size of the tumors reached 100mm³. *SHISA3* expression has been obtained by quantitative PCR and normalized by its expression in a control sample of Universal RNA.

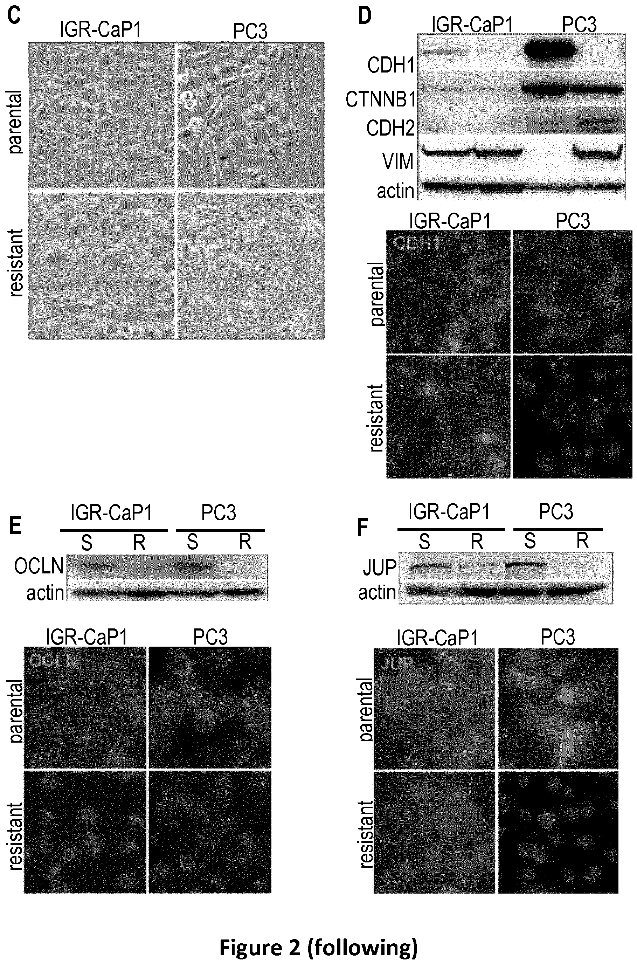

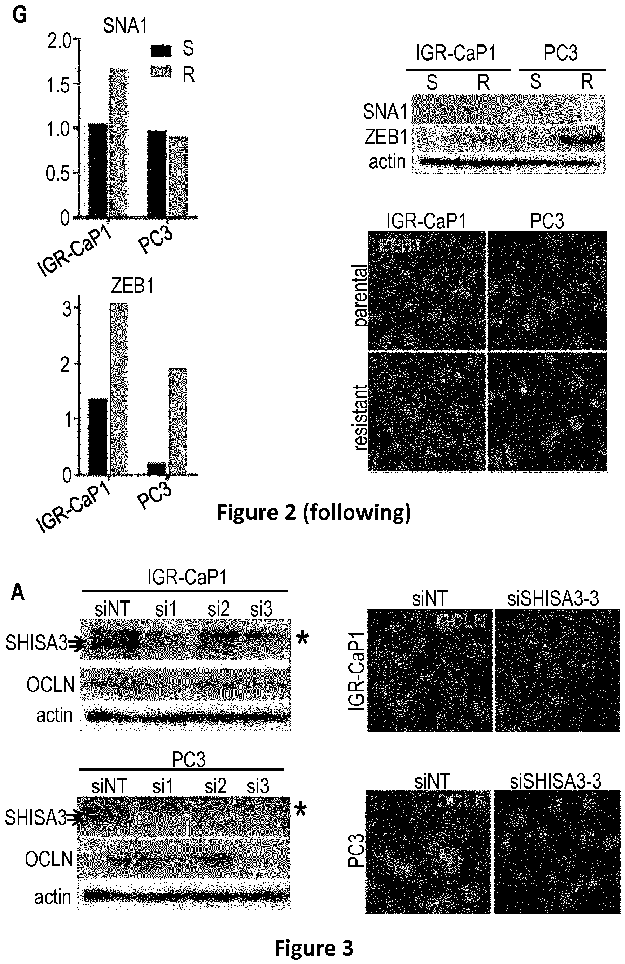

**Figure 2****: Characterization of EMT in docetaxel-Resistant cells compared to parental IGR-CaP1 and PC3 Cells**
   **A.** Heat map showing the miRNA signature (FC>1.5) obtained in Docetaxel-resistant cells overlapping with an EMT-associated miRNA signature (Diaz-Martin J et al, J Pathol, 2014, 232(3), pp. 319-29). **B.** Venn diagram showing genes with differential expression (FC>2) in Docetaxel-resistant IGR-CaP1 and PC3 cell lines compared with an EMT-associated gene signature (Gröger CJ, et al. PLoS One 2012; 7:e51136). **C.** Cell morphological difference between parental and Docetaxel-resistant cell lines in phase contrast microscopy. **D-F.** Expression of EMT markers in parental and Docetaxel-resistant IGR-CaP1 and PC3 cells by western-blot and immunofluorescence analyses: (**D**) Epithelial (E-cadherin/CDH1 and β-catenin/CTNNB1) and mesenchymal (N-cadherin/CDH2 and Vimentin/VIM) markers; (**E**) (**F**) Tight junction proteins Occludin (OCLN) and desmosome protein Plakoglobin (JUP), respectively. Immunofluorescence was performed on IGR-CaP1 and PC3 cells using anti-occludin and anti-plakoglobin antibodies and labeled with alexa-488 conjugated antibody. Nuclei were stained with Dapi. **G.** Expression of EMT transcription factors Snail/SNAI1 and ZEB1 in parental and resistant cells determined by qRT-PCR, western blot, and immunofluorescence analyses using anti-ZEBl antibodies. Nuclei were stained with Dapi.

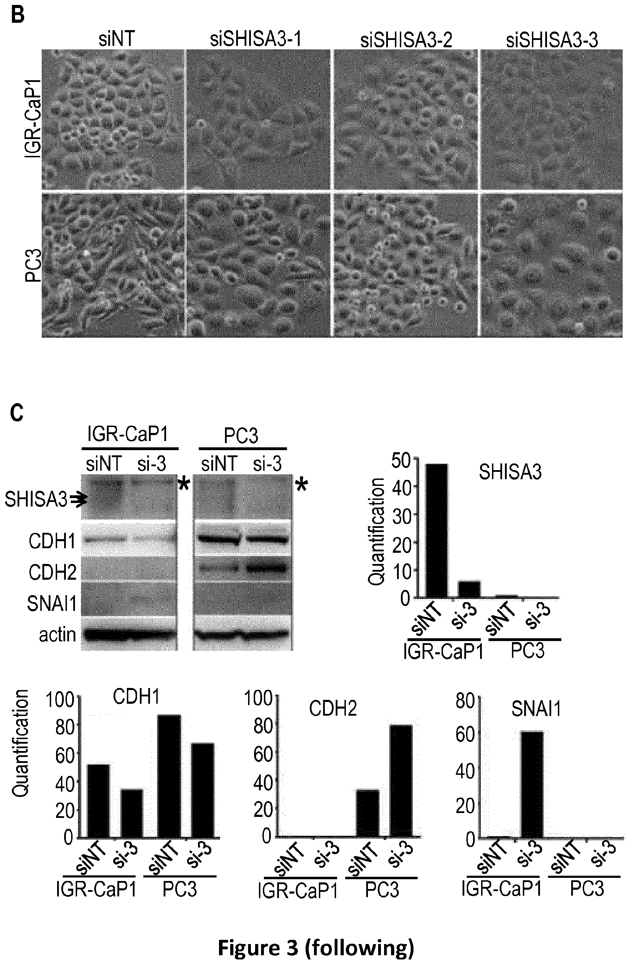

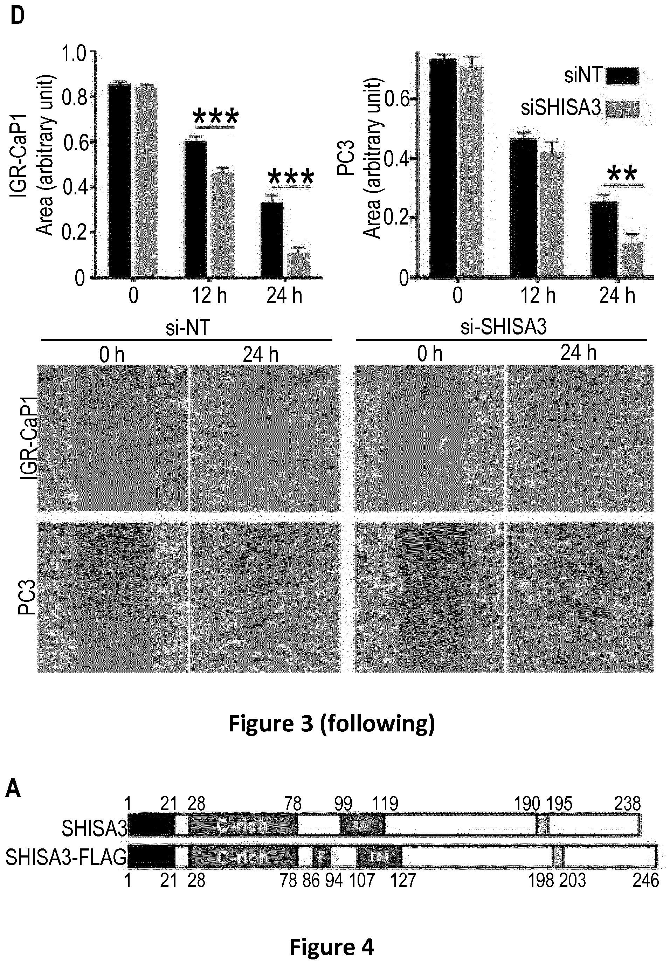

**Figure 3****: Silencing of SHISA3 induces EMT in IGR-CaP1 and PC3 parental cells**
   **A.** *SHISA3* depletion with siRNA leads to the loss of tight junction protein Occludin. Cells transfected for 5 days with either a non-targeted siRNA (siNT) or with different siRNA targeting *SHISA3* and were analyzed by western-blot and immunofluorescence. Western-blot analysis was performed using specific antibodies for *SHISA3,* Occludin (OCLN) or actin. **B.** Cell morphological changes observed 5 days after transfection of non-targeted siRNA (siNT) or different specific *SHISA3* siRNAs. **C.** *SHISA3* siRNA decreases the expression of the epithelial E-cadherin (CDH1) and increases the expression of N-cadherin (CDH2) and snail (SNAI1) as determined by immunoblot. Quantification of bands relative to actin is shown. **D.** Cell migration was measured in wound healing assays after 5 days with siSHISA3 or siNT. The area between the wound edges was quantified by Image J at 0, 12, and 24 hours after wound healing. Bars represent the mean ± SEM (n=14). P value was derived from the two-tailed Student's t test. (p <0.05 : * ; p <0.005 : ** ; p <0.0005 : ***).

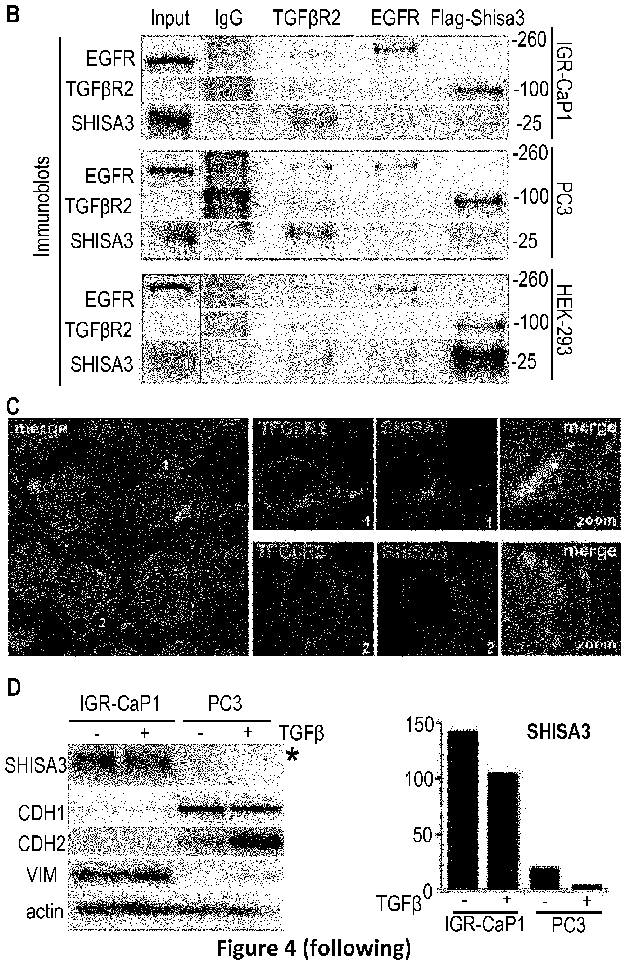

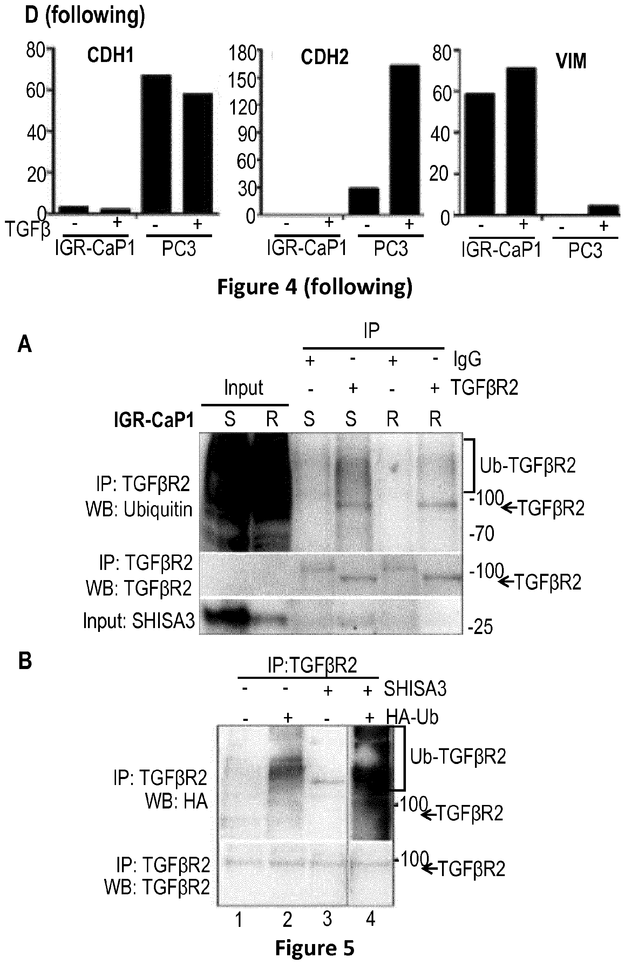

**Figure 4****: SHISA3 interacts with TGFβR2**
   **A.** Schematic structure of *SHISA3* and *SHISA3-Flag* sharing a signal peptide (black box), an amino-terminal CRD (blue box), a predicted transmembrane segment (TM), and a carboxy-terminal proline-rich region (Grey box). The position of the FLAG tag (red box) is indicated. **B.** SHISA3 and endogenous TGFβR2 interaction. Cells extracts from IGR-CaP1, PC3 and HEK cells transfected with *SHISA3-Flag* were immunoprecipitated with anti-TGFβR2, anti-EGFR, anti-Flag antibodies or IgG as control and analyzed by immunoblot using anti-TGFβR2, anti-EGFR and anti-SHISA3 antibodies. Input represents 10% of the IP. **C.** Co-localization of SHISA3 and TGFβR2 in cytoplasmic granules and speckles at the membrane. Subcellular localizations of SHISA3 and TGFβR2 were analyzed by confocal microscopy after co-transfection of *SHISA3-Flag* and *HA-TGFβR2* in HEK cells using anti-SHISA3 and anti-TGFβR2 primary antibodies, and Alexa-568- and Alexa-647-conjugated secondary antibodies respectively. Nuclei were labeled with Dapi. Overlapped fluorescent signals (yellow) indicated co-localized proteins. **D.** SHISA3 and EMT markers expression 5 days after cell treatment with TGFβ1. Whole cells extracts of cells treated with mock or TGFβ were subjected to immunoblot and revealed with anti-SHISA3 and EMT marker antibodies. Quantification of bands relative to beta actin is shown.
**Figure 5****: SHISA3 increases TGFβR2 ubiquitination**
   **A.** Immunoprecipitated forms of endogenous TGFβR2 were highly ubiquitinated in SHISA3-expressing parental IGR-CaP1 cells. Immunoprecipitated proteins with the anti-TGFβR2 antibody or IgG as control were analyzed by immunoblotting with anti-TGFβR2 and anti-ubiquitin antibodies. Input represents 10% of immunoprecipitated proteins. Expression of co-immunoprecipitated SHISA3 is shown. **B.** SHISA3 increases ubiquitination of endogenous TGFβR2. IGR-CaP1 cells were transfected with SHISA-Flag and HA-Ubiquitine expressing vectors or corresponding empty vectors. Cellular extracts were prepared 48h after transfection and proteins were immunoprecipitated with anti-TGFβR2 antibody. Immunoblots were revealed with anti-TGFβR2 and anti-HA antibodies.

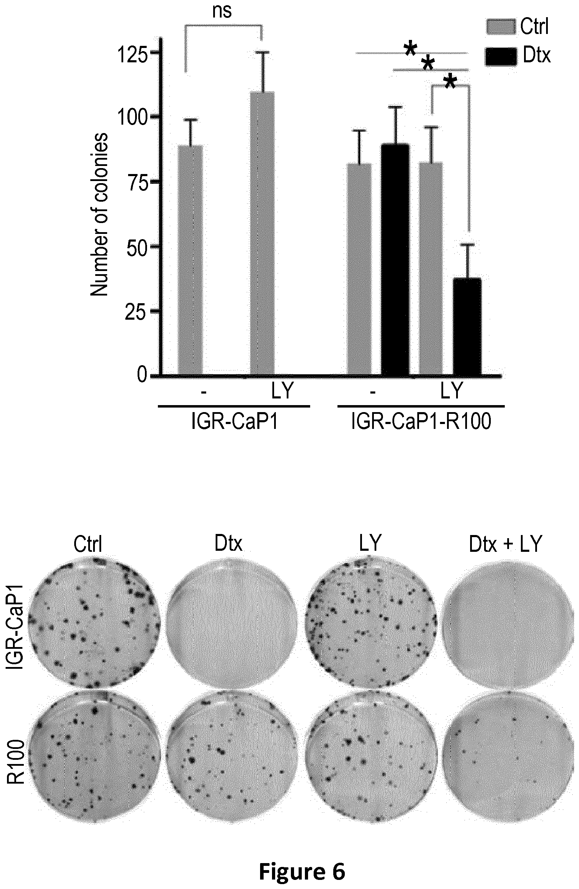

**Figure 6****: Targeting the TGFβ signaling with a small inhibitor increases cell death of docetaxel resistant cells**
   Colony formation assay. Cells were pretreated with 25µM LY2157299 or vehicle for 72 hours. Then, cells were treated with Docetaxel and/or 25µM LY2157299, control cells were treated with vehicle. Docetaxel was used at 12nM in parental IGR-CaP1 and 100nM in resistant IGR-CaP1-R100. Cells were stained with Crystal Violet 2 weeks later. Data are represented as mean ± SD. *P* value was derived from the two-tailed Student's *t* test, significantly different (**P*<0.05,). Representative cell colonies obtained in each condition is shown.

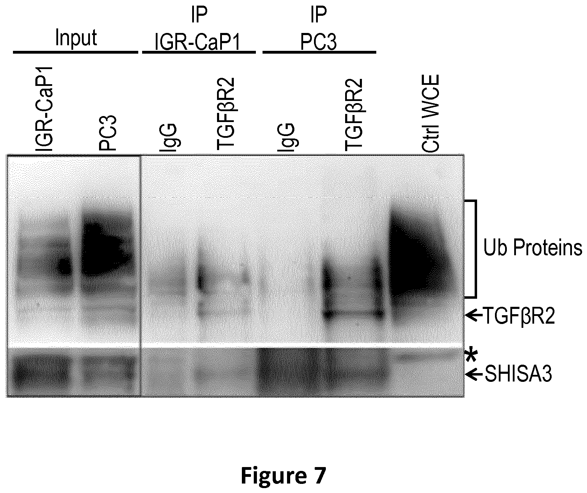

**Figure 7****: Immunoprecipitation of endogenous TGFβR2 in IGR-CaP1 and PC3 cells**
   Endogenous TFGβR2 from IGR-CaP1 and PC3 cells were immunoprecipitated with anti-TGFβR2 antibody or IgG as control. Immunoblots of immunoprecipitated proteins were revealed with anti-TGFβR2 antibody and with anti-ubiquitin antibody to observe the poly-ubiquitinated forms of the receptor. A loading control of whole cell extracts of cells treated with MG132 was used as control of ubiquitinated proteins. Input represents 10% of the IP. Expression of co-immunoprecipitated SHISA3 is shown. Star represents a non-specific band.

### DETAILED DESCRIPTION OF THE INVENTION

The present invention relates to a new method for selecting patients susceptible to benefit from a new combined treatment aimed to overcome cancer taxoid resistance. Nowadays, taxoid are widely used as cancer chemotherapy agents but, unfortunately, development of resistances is also widely widespread, which is particularly dramatic since taxoids are often the last line of treatment available. Therapeutic strategies used so far to overcome docetaxel resistance have proved inefficient as evidence by reported negative phase III clinical trials combining new agents with docetaxel (Yap TA et al, Nat Rev Clin Oncol, 2011, 8(10), pp. 597-610; Corcoran NM et al, Histopathology, 2012, 60(1), pp. 216-31).

The inventors provide new evidences that EMT is a mechanism by which tumour cells acquire docetaxel resistance. In addition, they show that loss of SHISA3 is an early event in the initiation of the EMT process. In particular, SHISA3 loss induces E-cadherin loss, Snail transcription factor nuclear accumulation and enhances expression of N-cadherin, key characteristic features of EMT transition. Surprisingly, it was also shown that SHISA3 loss induced EMT was mediated by the TGF-β signalling pathway. The inventors further demonstrate that SHISA3 regulates the TGF-β signalling pathway by interacting with the TGF-β receptor 2 (TGF-βR2). In cancer cells, TGFβ binding to the membranous TGFβR2 receptor homodimer allows the dimerization of TGF-βR2 with TGF-βR1 receptor homodimers, then leading to activation of TGFβR1 kinase and subsequent intracellular signalling cascades through activation of the SMAD (Sma (small body size) and Mad (mother against decapentaplegic) related proteins) associated receptor/SMAD proteins pathway and inhibition of the GSK3β (glycogen synthase kinase-3β/SNAIL pathway (Xu J et al, Cell Res, 2009, 19(2), pp. 156-72). The inventors discovered that the interaction between SHISA3 and TGF-βR2 provokes endocytosis and ubiquitinylation of TGF-βR2, resulting in a disruption of the TGF-β signalling pathway. Accordingly, SHISA3 under-expression in prostate cancer cells (IGR-CaP1 (WO2010119001) and PC3 models) mediates a TGF-β induced EMT leading to the development of a chemoresistance to docetaxel. The inventors have yet demonstrated that targeting the TGFβ signalling pathway with inhibitors of the TGF-βR1, such as LY2157299, provokes, when combined with docetaxel, the death of docetaxel resistant cancer cells, thus reversing the docetaxel resistance.

This therapeutic strategy point out the need for a selection of patients eligible for a treatment combining a taxoid and a TGF-β signalling pathway inhibitor. According to above mentioned results, SHISA3 loss of expression allows to identify docetaxel-resistant patients that will benefit from this combined treatment.

The methods of the invention, as disclosed below, may be *in vivo, ex vivo* or *in vitro* methods, preferably *in vitro* methods.

The present invention concerns, in a first aspect, a method for selecting a patient affected with a tumor for a treatment with a molecule of the taxoid family in combination with a compound inhibiting the TGF-β signaling pathway or determining whether a patient affected with a tumor is susceptible to benefit from a treatment with a molecule of the taxoid family in combination with a compound inhibiting the TGF-β signaling pathway, wherein the method comprises determining the expression level of SHISA3 in a cancer sample from said patient, comparing the expression level of SHISA3 to a reference expression level, wherein the under-expression of SHISA3 is predictive that a treatment with a molecule of the taxoid family in combination with a compound inhibiting the TGF-β signaling pathway is indicated for said patient and optionally, selecting patients with under-expression of SHISA3 for a treatment with a molecule of the taxoid family in combination with a compound inhibiting the TGF-β signalling pathway. Optionally, the method further comprises a previous step of providing a cancer sample from said patient.

As used herein, the term "SHISA3" refers to the product of the human gene LOC152573, also named *Shisa3.* This protein was formerly named "hypothetical protein BC012029" (UniProt accession number: AOPJX4). The human gene *Shisa3* (UniGene Hs.370904; GeneID: 152573) is located on chromosome 4 in location 4p13. The NCBI accession number of the sequence of the mRNA of the human SHISA3 protein is NM_001080505. The NCBI accession number of the sequence of the precursor of the human SHISA3 protein is NP_001073974.

The expression level of SHISA3 can be determined by a variety of techniques well known by the skilled person. In an embodiment, the expression level of SHISA3 is determined by measuring the quantity of SHISA3 protein or SHISA3 mRNA.

In a particular embodiment, the expression level of SHISA3 is determined by measuring the quantity of SHISA3 protein. The quantity of SHISA3 protein may be measured by any methods known by the skilled person. Usually, these methods comprise contacting the sample with a binding partner capable of selectively interacting with the SHISA3 protein present in the sample. The binding partner is generally a polyclonal or monoclonal antibody, preferably monoclonal. Such an antibody can be produced through methods known to the man skilled in the art. This antibody includes in particular those produced by a hybridoma and those produced by genetic engineering using host cells transformed with a recombinant expression vector carrying a gene encoding the antibody. A hybridoma producing monoclonal antibodies can be obtained as following: SHISA3 protein or immunogenic fragments thereof are used as antigen for immunisation according to conventional methods of immunisation. The resulting immunocytes are fused with known parent cells according to conventional cell fusion methods and the cells producing the antibodies are thus screened from fused cells by conventional screening methods. The invention concerns an antibody specific of human SHISA3 or fragment thereof.

The quantity of SHISA3 protein may be measured by semi-quantitative Western blots, enzyme-labeled and mediated immunoassays, such as ELISAs, biotin/avidin type assays, radioimmunoassay, immunoelectrophoresis or immunoprecipitation or by protein or antibody arrays. The protein expression level may be assessed by immunohistochemistry. The reactions generally include revealing labels such as fluorescent, chemiluminescent, radioactive, enzymatic labels or dye molecules, or other methods for detecting the formation of a complex between the antigen and the antibody or antibodies reacted therewith.

In another particular embodiment, the expression level of SHISA3 is determined by measuring the quantity of SHISA3 mRNA. Methods for determining the quantity of mRNA are well known in the art. mRNA can be detected by hybridization (e. g., Northern blot analysis) and/or amplification (e.g., RT-PCR). Preferably, mRNA is detected by quantitative or semi-quantitative RT-PCR. Real-time quantitative or semi-quantitative RT-PCR is particularly advantageous. Preferably, primer pairs were designed in order to overlap an intron. Other primers may be easily designed by the skilled person. Taqman probes specific of the SHISA3 transcript may be used (e.g. Taqman probe of Applied Biosystem with assay ID :Hs01380806_m1). Other methods of Amplification include ligase chain reaction (LCR), transcription-mediated amplification (TMA), strand displacement amplification (SDA) and nucleic acid sequence based amplification (NASBA).

In a preferred embodiment, the expression level of SHISA3 is determined by measuring the quantity of SHISA3 mRNA, preferably by quantitative or semi-quantitative RT-PCR or by real-time quantitative or semi-quantitative RT-PCR.

In another embodiment, the "expression level of SHISA3" may be understood as the quantification of the amount of SHISA3 interacting with TGF-βR2. In particular, this amount may be determined by a proximity ligation assay (PLA). A kit is commercially available for PLA (DUOLink kit (OLINK, Uppsala, Sweden).

Proximity ligation assays (PLAs) can be performed on both fixed and permeabilized transfected cell lines. According to the manufacturer's instructions (Olink ioscience, Uppsala,Sweden), a first step of incubation with the primary antibodies at 4°C overnight is followed by blocking (OLINK). Then, proximity ligation assay minus and plus probes PLA (containing the secondary antibodies conjugated with oligonucleotides) are added and incubated 1 h at 37°C. Afterwards, further oligonucleotides are added, allowed to hybridize to the PLA probes, and ligase joins the two hybridized oligonucleotides to a closed circle. The DNA is then amplified (rolling circle amplification), and detection of amplicons is carried out using the 'Brigthfield detection kit' for the chromogenic revelation or using the 'far Red detection Kit' for the fluorescence revelation. The number of in situ PLA signals per cell was counted by semi-automated image analysis.

As used herein, the term "molecule of the taxoid family" refers to a molecule belonging to the anti-tumoral drug taxane family. Molecules of the taxane family bind to β-tubulin and stabilize the polymerized form of the microtubule. Thus, they act as mitotic inhibitors by disrupting the microtubule function. The molecule of the taxoid family can be selected from the group consisting of paclitaxel, docetaxel and analogs, prodrugs or formulations thereof. In particular, the molecule of the taxoid family can be selected in the group consisting of paclitaxel (also called taxol®), docetaxel (also called taxotere®; Sanofi-Aventis), larotaxel (also called XRP9881; Sanofi-Aventis), cabazitaxel (also called XRP6258; Sanofi-Aventis), BMS-184476 (Bristol-Meyer-Squibb), BMS-188797 (Bristol-Meyer-Squibb), BMS-275183 (Bristol-Meyer-Squibb), ortataxel (also called IDN 5109, BAY 59-8862 or SB-T-101131 ; Bristol-Meyer-Squibb), RPR 109881A (Bristol-Meyer-Squibb), RPR 116258 (Bristol-Meyer-Squibb), NBT-287 (TAPESTRY), PG-paclitaxel (also called CT-2103, PPX, paclitaxel poliglumex, paclitaxel polyglutamate or Xyotax™), ABRAXANE® (also called Nab-Paclitaxel; ABRAXIS BIOSCIENCE), Tesetaxel (also called DJ-927), IDN 5390 (INDENA), Taxoprexin (also called docosahexanoic acid-paclitaxel ; PROTARGA), DHA-paclitaxel (also called Taxoprexin®), and MAC-321 (WYETH), Genexol-PM (Samyang Biopharmaceuticals Corporation), LEP (Liposomal-encapsulated paclitaxel or LEP-ETU; NeoPharm), Tocosol® paclitaxel (Sonus Pharmaceutical), SB-T-1213, SB-T-1214, SB-T-1216, SB-T-1217, SB-T-121303, SB-T-121303021, SB-T-12130301, and a combination thereof. Also see the review of Hennenfent & Govindan (2006, Annals of Oncology, 17, 735-749). Preferably, the molecule of the taxoid family is selected in the group consisting of docetaxel (taxotere®), larotaxel, cabazitaxel (XRP6258), BMS-184476, BMS-188797, BMS-275183, ortataxel, RPR 109881A, RPR 116258, NBT-287, PG-paclitaxel, ABRAXANE®, Tesetaxel, IDN 5390, Taxoprexin, DHA-paclitaxel, MAC-321, Genexol-PM, LEP, Tocosol® paclitaxel, SB-T-1213, SB-T-1214, SB-T-1216, SB-T-1217, SB-T-121303, SB-T-121303021, SB-T-12130301 and a combination thereof. In a preferred embodiment of the present invention, the molecule of the taxoid family is the docetaxel.

As used herein, the "compound inhibiting the TGF-β signalling pathway" may be any compound which is able to decrease the activity of the TGF-β (transforming growth factor β) signalling pathway. Such a compound may be identified by screening methods as disclosed below. The capacity of a compound to decrease the activity of the TGF-β signalling pathway can be assessed by contacting a cell-line in which SHISA3 is under-expressed in comparison to a reference expression level with the compound to be tested and determining the activity level of the TGF-β signaling pathway. In addition, kits for testing TGF-βP activity are commercially available and method for testing the activity of TGF-βP are well-known in the art.

As used herein, the term "Transforming growth factor-beta receptor type I (TGF-βR1)" is equivalent to "Activin receptor-like kinase 5 (ALK5)", "Activin A receptor type II like kinase (ACVRLK-4)", or "Serine/threonine-protein kinase receptor 4 (SKR4)". As used herein, the term "Transforming growth factor-beta receptor type II (TOPP-βP2) is equivalent to "Activin receptor-like kinase 7 (ALK7)", "Marfan syndrome, type II (MFS2)".

The compound inhibiting the TGF-β signalling pathway can act at different levels of this signalling pathway. This compound can prevents the synthesis of TGF-β, provokes its degradation or prevents its fixation to its receptor. This compound can also prevents the synthesis of the TOPP-βR1 and/or the TOPP-βR2, provokes their internalisation and/or their degradation, or inhibits their activity. Moreover, this compound can prevents the synthesis, provokes the degradation or prevents the action of a protein of an intracellular signalling cascade activated by the TGF-β signalling pathway such as SMAD associated receptor or SMAD proteins. Finally, this compound can enhances the synthesis, prevents the degradation or enhances the action of a protein of an intracellular signalling cascade inhibited by the TGF-β signalling pathway such as GSK3β or SNAIL. Also see the review of Connolly EC et al. (2012, International Journal of Biological Sciences, 8 (7), pp. 964-978). Others inhibitors of the TGF-β signalling pathway are also disclosed in WO 99/01765, WO 03/093293, WO 03/061587, WO 03/070197, WO 2004/037209, WO 2005/007199, WO 2005/037221, WO 2007079820, WO 2007/143023, WO 2009/146408, WO 2010/012345, WO 2012/000595, WO 2012/003912, WO 2012/11960).

In a particular embodiment, the compound inhibiting the TGF-β signaling pathway is an inhibitor of TGF-β synthesis. The inhibitor of TGF-β synthesis can be selected in the group consisting of Pirfenidone (InterMune), antisense oligonucleotides directed against TGF-β ARNm like trabedersen (also called AP12009, Antisense Pharma), a RNAi interfering specifically with TGF-β expression, thereby decreasing or suppressing the expression of this protein. By "decreasing the expression", it is meant, for example, a decrease of 30%, 50%, 70%, 80%, 90% or 95% of the gene expression product.

Antisense nucleic acid can be used to down-regulate the expression of TGF-β. The antisense nucleic acid can be complementary to all or part of a sense nucleic acid encoding TGF-β e.g., complementary to the coding strand of a double-stranded cDNA molecule or complementary to an mRNA sequence, and is thought to interfere with the translation of the target mRNA. Preferably, the antisense nucleic acid is an RNA molecule complementary to a target mRNA encoding TGF-β. An antisense nucleic acid can be, for example, about 5, 10, 15, 20, 25, 30, 35, 40, 45 or 50 nucleotides in length. Particularly, antisense RNA molecules are usually 18-50 nucleotides in length. An antisense nucleic acid can be constructed using chemical synthesis and enzymatic ligation reactions using procedures known in the art. Particularly, antisense RNA can be chemically synthesized, produced by *in vitro* transcription from linear (e.g. PCR products) or circular templates (e.g., viral or non-viral vectors), or produced by *in vivo* transcription from viral or non-viral vectors. Antisense nucleic acid may be modified to have enhanced stability, nuclease resistance, target specificity and improved pharmacological properties. For example, antisense nucleic acid may include modified nucleotides designed to increase the physical stability of the duplex formed between the antisense and sense nucleic acids, e.g., phosphorothioate derivatives and acridine substituted nucleotides.

The term "RNAi" or "interfering RNA" means any RNA which is capable of down-regulating the expression of the targeted protein. It encompasses small interfering RNA (siRNA), double-stranded RNA (dsRNA), single-stranded RNA (ssRNA), micro-RNA (miRNA), and short hairpin RNA (shRNA) molecules. RNA interference, designate a phenomenon by which dsRNA specifically suppresses expression of a target gene at post-translational level. In normal conditions, RNA interference is initiated by double-stranded RNA molecules (dsRNA) of several thousands of base pair length. In vivo, dsRNA introduced into a cell is cleaved into a mixture of short dsRNA molecules called siRNA. The enzyme that catalyzes the cleavage, Dicer, is an endo-RNase that contains RNase III domains (Bernstein et al., 2001). In mammalian cells, the siRNAs produced by Dicer are 21-23 bp in length, with a 19 or 20 nucleotides duplex sequence, two-nucleotide 3' overhangs and 5'-triphosphate extremities (Elbashir et al., 2001a; Elbashir et al., 2001b; Zamore et al., 2000). A number of patents and patent applications have described, in general terms, the use of siRNA molecules to inhibit gene expression, for example, WO 99/32619, US 20040053876, US 20040102408 and WO 2004/007718.

siRNA are usually designed against a region 50-100 nucleotides downstream the translation initiator codon, whereas 5'UTR (untranslated region) and 3'UTR are usually avoided. The chosen siRNA target sequence should be subjected to a BLAST search against EST database to ensure that the only desired gene is targeted. Various products are commercially available to aid in the preparation and use of siRNA. In a preferred embodiment, the RNAi molecule is a siRNA of at least about 15-50 nucleotides in length, preferably about 20-30 base nucleotides.

RNAi can comprise naturally occurring RNA, synthetic RNA, or recombinantly produced RNA, as well as altered RNA that differs from naturally-occurring RNA by the addition, deletion, substitution and/or alteration of one or more nucleotides. Such alterations can include addition of non-nucleotide material, such as to the end of the molecule or to one or more internal nucleotides of the RNAi, including modifications that make the RNAi resistant to nuclease digestion.

RNAi may be administered in free (naked) form or by the use of delivery systems that enhance stability and/or targeting, e.g., liposomes, or incorporated into other vehicles, such as hydrogels, cyclodextrins, biodegradable nanocapsules, bioadhesive microspheres, or proteinaceous vectors (WO 00/53722), or in combination with a cationic peptide (US 2007275923). They may also be administered in the form of their precursors or encoding DNAs.

In a particular embodiment, the compound inhibiting the TGF-β signaling pathway is an inhibitor of TGF-βR I or II, Smad receptor or Smad proteins synthesis. This inhibitor can be an antisense oligonucleotide or a RNAi as disclosed above.

In another embodiment, the compound inhibiting the TGF-β signaling pathway is a ligand trap such as anti-TGF-β antibodies, recombinant proteins capable to bind TGF-β, in particular, recombinant proteins containing the soluble ectodomain of the TGF-βRI, TGF-βRII or TGF-βRIII, TGF-β binding peptides such as P144 (Sigma-Genosys), or aptamers directed against TGF-β.

An anti-TGF-β antibody can be produced through methods known to the man skilled in the art. This antibody includes in particular those produced by a hybridoma and those produced by genetic engineering using host cells transformed with a recombinant expression vector carrying a gene encoding the antibody. A hybridoma producing monoclonal antibodies can be obtained as following: TGF-β protein or immunogenic fragments thereof are used as antigen for immunisation according to conventional methods of immunisation. The resulting immunocytes are fused with known parent cells according to conventional cell fusion methods and the cells producing the antibodies are thus screened from fused cells by conventional screening methods. The invention concerns an antibody specific of human TGF-β or fragment thereof. Anti-TGF-β antibody can be selected in the group consisting of 1D11 (Sanofi Aventis), fresolimumab (Sanofi Aventis), Lerdelimumab (Cambridge Antibody Technology) or metelimumab (Genzyme).

As used herein, the term 'recombinant protein' is equivalent to 'chimeric protein' and are proteins resulting from the expression of recombinant DNA. Recombinant DNA molecules are DNA molecules formed by genetic recombination (such as molecular cloning) to bring together genetic material from multiple sources, creating sequences that would not otherwise be found in biological organisms. DNA recombination techniques are well known to the man skilled in the art. In the present embodiment, recombinant proteins can be a combination of the ectodomain of a TGF-βR and a protein suitable for extracellular expression.

As used herein, the term "aptamer" means a molecule of nucleic acid or a peptide able to bind specifically to a protein. In a preferred embodiment, the aptamers are nucleic acids, preferably RNA, generally comprising between 5 and 120 nucleotides (Osborne *et al.,* 1997). They can be selected *in vitro* according to a process known as SELEX (Systematic Evolution of Ligands by Exponential Enrichment).

In yet another embodiment, the compound inhibiting the TGF-β signaling pathway is a neutralizing antibody targeting TGF-β receptors and preventing the liaison of TGF-β (Kasuga et al., Kidney Int, 2001, 60(5), pp. 1745-55).

An anti- TGF-β receptors antibody can be produced through methods known to the man skilled in the art and as disclosed above. As used herein, the term "neutralizing antibody targeting TGF-β receptors" designates an antibody as described above which is able to bind to the extracellular domain of targeting TGF-β receptors and to block or reduce their activity. This inhibition can be due to steric hindrance or modification which prevents ligand binding.

As used herein, the terms "antibody" refers to immunoglobulin molecules and immunologically active portions of immunoglobulin molecules, i.e., molecules that contain an antigen-binding site that immunospecifically binds an antigen. As such, the term antibody encompasses not only whole antibody molecules, but also antigen-binding antibody fragments as well as variants (including derivatives) of antibodies and antibody fragments. In particular, the antibody according to the invention may correspond to a polyclonal antibody, a monoclonal antibody (e.g. a chimeric, humanized or human antibody), a fragment of a polyclonal or monoclonal antibody or a diabody.

In natural antibodies, two heavy chains are linked to each other by disulfide bonds and each heavy chain is linked to a light chain by a disulfide bond. Each chain contains distinct sequence domains. The light chain includes two domains, a variable domain (V_{L}) and a constant domain (C_{L}). The heavy chain includes four domains, a variable domain (V_{H}) and three constant domains (C_{H}1, C_{H}2 and C_{H}3, collectively referred to as C_{H}). The variable regions of both light (V_{L}) and heavy (V_{H}) chains determine binding recognition and specificity to the antigen. Are also contemplated camelid antibodies, such as heavy-chain antibodies, and fragments and derivatives thereof such (VHH)₂ fragments, nanobodies and sdAb.

The specificity of the antibody resides in the structural complementarity between the antibody combining site and the antigenic determinant. Antibody combining sites are made up of residues that are primarily from the hypervariable or complementarity determining regions (CDRs). They refer to amino acid sequences which, together, define the binding affinity and specificity of the natural Fv region of a native immunoglobulin binding site. The light and heavy chains of an immunoglobulin each have three CDRs, designated L-CDR1, L-CDR2, L-CDR3 and H-CDR1, H-CDR2, H-CDR3, respectively. Therefore, an antigen-binding site includes six CDRs, comprising the CDR set from each of a heavy and a light chain V region.

Framework regions (FRs) refer to amino acid sequences interposed between CDRs, i.e. to those portions of immunoglobulin light and heavy chain variable regions that are relatively conserved among different immunoglobulins in a single species, as defined by Kabat *et al.,* 1991 (Kabat et al., 1991, Sequences of Proteins Of Immunological Interest, National Institute of Health, Bethesda, Md). As used herein, a "human framework region" is a framework region that is substantially identical (about 85%, or more, in particular, 90%, 95% or 100%) to the framework region of naturally occurring human antibody.

The term "monoclonal antibody" or "mAb" as used herein refers to an antibody molecule of a single amino acid composition, that is directed against a specific antigen and which may be produced by a single clone of B cells or hybridoma, or by recombinant methods.

A "humanized antibody" is a chimeric, genetically engineered, antibody in which the CDRs from an antibody, e.g. a mouse antibody, ("donor antibody") are grafted onto a human antibody ("acceptor antibody"). Thus, a humanized antibody is an antibody having CDRs from a donor antibody and variable region framework and constant regions from a human antibody. The use of antibody components derived from humanized monoclonal antibodies obviates potential problems associated with the immunogenicity of murine constant regions.

Antibodies according to the invention may be produced by any technique known in the art, such as, without limitation, any chemical, biological, genetic or enzymatic technique, either alone or in combination. The antibodies of this invention can be obtained by producing and culturing hybridomas.

The term "Heavy-chain antibodies" or "HCAbs" refer to immunoglobulins which are devoid of light chains and consist in two heavy chains. These antibodies do not rely upon the association of heavy and light chain variable domains for the formation of the antigen-binding site but instead the variable domain of the heavy polypeptide chains alone naturally form the complete antigen binding site. Each heavy chain comprises a constant region (CH) and a variable domain which enables the binding to a specific antigen, epitope or ligand. As used herein, HCAbs encompass heavy chain antibodies of the camelid-type in which each heavy chain comprises a variable domain called VHH and two constant domains (CH2 and CH3). Such heavy-chain antibodies directed against a specific antigen can be obtained from immunized camelids. Camelids encompass dromedary, camel lama and alpaca. Camelid HCAbs have been described by Hamers-Casterman et al., 1993. Other examples of HCAb are immunoglobulin-like structures (Ig-NAR) from cartilaginous fishes. Heavy-chain antibodies can be humanized using well-known methods.

"Antibody fragments" comprise a portion of an intact antibody, preferably the antigen binding or variable region of the intact antibody. Examples of antibody fragments include Fv, Fab, F(ab')₂, Fab', Fd, dAb, dsFv, scFv, di-scFvs, sc(Fv)₂, CDRs, VHH, (VHH)₂, diabodies and multi-specific antibodies formed from antibodies fragments.

The term "Fab" denotes an antibody monovalent fragment having a molecular weight of about 50,000 and antigen binding activity, and consisting of the V_{L}, V_{H}, C_{L} and C_{H}1 domains which can be obtained by cutting a disulfide bond of the hinge region of the F(ab')₂ fragment.

The Fv fragment is the N-terminal part of the Fab fragment and consists of the variable portions of one light chain and one heavy chain.

The term "F(ab')₂" refers to an antibody bivalent fragment having a molecular weight of about 100,000 and antigen binding activity, which comprises two Fab fragments linked by a disulfide bridge at the hinge region.

The term "Fd" refers to an antibody fragment consisting of the V_{H} and C_{H}1 domains.

The term "dAb" (Ward et al., 1989 Nature 341:544-546) refers to a single variable domain antibody, i.e. an antibody fragment which consists of a V_{H} or V_{L} domain.

A single chain Fv ("scFv") polypeptide is a covalently linked V_{H}::V_{L} heterodimer which is usually expressed from a gene fusion including V_{H} and V_{L} encoding genes linked by a peptide-encoding linker. "dsFv" is a V_{H}::V_{L} heterodimer stabilised by a disulfide bond. Divalent and multivalent antibody fragments can form either spontaneously by association of monovalent scFvs such as di-scFvs, or can be generated by coupling monovalent scFvs by a peptide linker, such as divalent sc(Fv)₂.

The term "diabodies" refers to small antibody fragments with two antigen-binding sites, which fragments comprise a V_{H} domain connected to a V_{L} domain in the same polypeptide chain (V_{H}-V_{L}). By using a linker that is too short to allow pairing between the two domains on the same chain, the domains are forced to pair with the complementarity domains of another chain and create two antigen-binding sites. The diabody may be mono- or bi-specific.

The term "VHH" refers to an antibody fragment consisting of the V_{H} domain of camelid heavy-chain antibody. VHH fragments can be produced through recombinant DNA technology in a number of microbial hosts (bacterial, yeast, mould), as described in WO 94/29457. Alternatively, binding domains can be obtained by modification of the VH fragments of classical antibodies by a procedure termed "camelisation", described by Davies et al, 1995. Dimers of VHH fragments, i.e. (VHH)₂, can be generated by fusing two sequences encoding VHH fragments, end to end, e.g. by PCR. Preferably, the (VHH)₂ fragment is monospecific.

Rodent monoclonal antibodies to specific antigens may be obtained by methods known to those skilled in the art. (See, e.g., Kohler and Milstein, Nature 256: 495 (1975), and Coligan et al. (eds.), CURRENT PROTOCOLS IN IMMUNOLOGY, VOL. 1, pages 2.5.1-2.6.7 (John Wiley & Sons 1991)).

Antibody fragments which recognize specific epitopes can be generated by known techniques. The antibody fragments are antigen binding portions of an antibody, such as F(ab)2, Fab', Fab, Fv, scFv and the like. Other antibody fragments include, but are not limited to: the F(ab')2 fragments which can be produced by pepsin digestion of the antibody molecule and the Fab' fragments, which can be generated by reducing disulfide bridges of the F(ab')2 fragments. Alternatively, Fab' expression libraries can be constructed (Huse et al., 1989, Science, 246:1274-1281) to allow rapid and easy identification of monoclonal Fab' fragments with the desired specificity.

In a particular embodiment, dominant-negative forms of the TGF-β receptors can be used to neutralize the TGF-β signaling pathway (Gorelik and Flavell, Nat Med, 2001, 7(10), pp.1118-22).

A dominant negative form of a TGF-β receptor is a receptor still capable to bind TGF-β but lacking or presenting an altered functional domain that prevents it to initiate its intracellular normal cascade signalization pathway. For example, TGF-β receptor I or II which are no more capable of TGF-βRI/TGF-βRII dimerization or TGF-β receptor I or II lacking their kinase activity.

In a preferred embodiment, the compound inhibiting the TGF-β signaling pathway is an inhibitor of the TGF-β receptor, in particular an inhibitor of the TGF-β receptor I or II, preferably an inhibitor of the TGF-β receptor I (TGF-βRI). The inhibitor of the TGF-β receptor I can be selected in the group consisting of: LY2157299 (also called Galunisertib, Apexbio), SB431542 (Sigma-Aldrich), LY2109761 (Cayman Chemical), SB525334 (Sigma-Aldrich), SB505124 (Sigma-Aldrich), RepSox (Sigma-Aldrich), GW788388 (Tocris Bioscience), LY364947 (Sigma-Aldrich), A 83-01 (Tocris Bioscience), SD 208 (Tocris Bioscience), D 4476 (Cayman Chemical), TGF-β RI Kinase Inhibitor VIII (Santa Cruz Biotechnology), Ki268994 (Kirin Brewery Company). Preferably, the inhibitor of the TGF-PRI is LY2157299.

In a particular embodiment, activation of TGF-β receptor III with ligands such as peptides or overexpression of the TGF-β receptor III can be used to neutralize the TGF-β signaling pathway. Indeed, the TGF-β receptor III stimulation is known to inhibit TGF-β receptor I/II signaling (Sharifi N et al, 2007, Prostate, 67(3), pp. 301-11; Ajiboye S et al, 2010, BJU Int, 105(7), pp. 913-6).

In another embodiment, the compound inhibiting the TGF-β signaling pathway is a neutralizing antibody or an aptamer targerting the Smad proteins, such as Trx-SARA (Zhao et al, Mol Biol Cell, 2006, 17(9), pp. 3819-31) or the Smad receptor.

Neutralizing antibodies and aptamers can be produced through methods known to the man skilled in the art and as disclosed above.

In yet another embodiment, the compound inhibiting the TGF-β signaling pathway is a dominant-negative form of Smad proteins such as Smad2 or Smad3 that bind the receptor but cannot be phosphorylated (Macias-Silva et al., Cell, 1996, 87(7): 1215-24).

A dominant negative form of a Smad protein is a Smad protein still capable to bind TGF-βR but lacking or presenting an altered domain that prevents it to be phosphorylated by TGF-β receptors.

The term "cancer" or "tumor", as used herein, refers to the presence of cells possessing characteristics typical of cancer-causing cells, such as uncontrolled proliferation, immortality, metastatic potential, rapid growth and proliferation rate, and certain characteristic morphological features. This term refers to any type of malignancy (primary or metastases). Preferably the cancer is a solid cancer. The cancer can be selected from the group consisting of the prostate cancer, the lung cancer, the breast cancer, the gastric cancer, the kidney cancer, the ovarian cancer, the hepatocellular cancer, the osteosarcoma, the melanoma, the hypopharynx cancer, the esophageal cancer, the endometrial cancer, the cervical cancer, the pancreatic cancer, the liver cancer, the brain cancer, the neuroendocrine tumors, the malignant tumor of the muscle, the adrenal cancer, the thyroid cancer, the uterine cancer, the skin cancer, the bladder cancer, and the head and neck cancer. In a preferred embodiment, the cancer is the prostate cancer.

The cancer treated by the combination of a molecule of the taxoid family and a compound inhibiting the TGF-β signalling pathway is preferably a cancer in which SHISA3 is under-expressed.

More preferably, the cancer treated by the combination of a molecule of the taxoid family and a compound inhibiting the TGF-β signalling pathway is further resistant to a molecule of the taxoid family, even more preferably to docetaxel.

Alternatively, the cancer treated by the combination of a molecule of the taxoid family and a compound inhibiting the TGF-β signalling pathway is a castration resistant prostate cancer.

In an embodiment, the cancer treated by the combination of a molecule of the taxoid family and a compound inhibiting the TGF-β signalling pathway is a cancer in which SHISA3 is under-expressed. Such a cancer is identified by comparing the expression level of SHISA3 in said cancer with a reference expression level. The reference expression level can be the expression level of SHISA3 in cell lines or tumors which are known to be sensitive to a treatment by the molecule of the taxoid family. Preferably, the cell line used to determine the reference expression level is a cancer cell line. More preferably, the cell line used to determine the reference expression level is a cell line providing from a cancer of the same type than the cancer to be treated. For instance, if the cancer to be treated is a prostate cancer, the cell line used to determine the reference expression level is a prostate cancer cell line. Even more preferably, the cell line used to determine the reference expression level is the IGR-CaP1 cell line (WO2010119001).

Alternatively, the reference expression level is the expression level of SHISA3 in a normal cell, i.e. a healthy cell. Preferably, the normal cell is histologically matched with the tumor. Optionally, the normal cell is from the same patient.

Optionally, before to be compared with the reference expression level, the expression levels may be normalized using the expression level of an endogenous control gene having a stable expression in different cancer samples, such as *RPLPO, HPRT1, GAPDH, B2M, TBP* and 18S genes.

SHISA3 is considered as under-expressed if the expression level in the tumor of the patient is, after normalization, at least 20, 30, 40, 50, 60, 70, 80 or 90 % lower than the reference expression level. In a preferred embodiment, the cancer treated by the combination of the compound increasing the expression level of SHISA3 and the molecule of the taxoid family is a cancer in which the expression level of SHISA3 is at least 50 % lower than the reference expression level.

In another embodiment, the cancer treated by the combination of a molecule of the taxoid family and a compound inhibiting the TGF-β signalling pathway is a cancer resistant to a molecule of the taxoid family. The term "cancer resistant to a molecule of the taxoid family" is intended to refer to a cancer that exhibits an inherent or acquired resistance to a treatment with a molecule of the taxoid family. A resistant cancer can exhibit a reduced sensitivity or even a full lack of sensitivity to a molecule of the taxoid family. When the resistance is acquired during therapy, it often manifests by a diminished amount of tumor regression, or even a tumor progression, for the same dose or by an increased dose which is necessary for tumor regression or for avoiding tumor progression. The sensitivity of a cancer to a treatment can be assessed by comparing the sensitivity of said cancer to the sensitivity of cancer cells which are known to be sensitive to said treatment and are thus responsive to the therapeutically effective dosage of a molecule of the taxoid family. The person skilled in the art has a lot of clinical parameters at his disposal to evaluate the sensitivity of a tumor to chemotherapy such as PSA progression, radiologic progression, pain progression etc.

In a particular embodiment, the cancer treated by the combination of a molecule of the taxoid family and a compound inhibiting the TGF-β signalling pathway is a cancer in which SHISA3 is under-expressed and is resistant to a molecule of the taxoid family.

As used herein, the term "treatment", "treat" or "treating" refers to any act intended to ameliorate the health status of patients such as therapy, prevention, prophylaxis and retardation of the disease. In certain embodiments, such term refers to the amelioration or eradication of a disease or symptoms associated with a disease. In other embodiments, this term refers to minimizing the spread or worsening of the disease resulting from the administration of one or more therapeutic agents to a subject with such a disease. In particular, the term "to treat a cancer", "treating a cancer", "to treat a tumor" or "treating a tumor" means reversing, alleviating, inhibiting the progress of, or preventing, either partially or completely, the growth of tumors, tumor metastases, or other cancer-causing or neoplastic cells in a patient.

The term "cancer sample" refers to any sample containing tumoral cells derived from the patient. In particular, tumoral cells may be obtained from fluid sample such as blood, plasma, urine and seminal fluid samples as well as from biopsies, organs, tissues or cell samples. In a preferred embodiment, tumoral cells are obtained from tumor biopsy or resection sample from the patient. Preferably, the sample contains only tumoral cells. Optionally, samples containing tumoral cells may be treated prior to their use. As example, a tumor cell enrichment sorting may be performed.

As used herein, the term "patient" refers to an animal, preferably to a mammal, more preferably to a human, including adult, child and human at the prenatal stage.

In a particular embodiment, the patient is affected with a prostate cancer and the molecule of the taxoid family is docetaxel.

In a particular aspect, the present invention concerns a method for selecting a patient affected with a tumor for a treatment with a molecule of the taxoid family or with a molecule of the taxoid family in combination with a compound inhibiting the TGF-β signaling pathway or determining whether a patient affected with a tumor is susceptible to benefit from a treatment with a molecule of the taxoid family or with a molecule of the taxoid family in combination with a compound inhibiting the TGF-β signaling pathway, wherein the method comprises providing a cancer sample from said patient; determining the expression level of SHISA3 in said sample; and comparing the expression level of SHISA3 to a reference expression level, wherein the normal or over-expression of SHISA3 is predictive that a treatment with a molecule of the taxoid family is indicated for said patient and wherein the under-expression of SHISA3 is predictive that a treatment with a molecule of the taxoid family in combination with a compound inhibiting the TGF-β signaling pathway is indicated for said patient, and optionally selecting patients with normal or over-expression of SHISA3 for a treatment with a molecule of the taxoid family and/or selecting patients with under-expression of SHISA3 for a treatment with a molecule of the taxoid family in combination with a compound inhibiting the TGF-β signalling pathway. Optionally, the method may further comprise a step of administering a therapeutically effective amount of a molecule of the taxoid family or a molecule of the taxoid family in combination with a compound inhibiting the TGF-β signaling pathway.

By a "therapeutically effective amount" is intended an amount of therapeutic agent, a molecule of the taxoid family, a compound inhibiting the TGF-β signalling pathway or a combination of a molecule of the taxoid family and a compound inhibiting the TGF-β signalling pathway, administered to a patient that is sufficient to constitute a treatment of taxoid resistant cancer under-expressing SHISA3.

In another particular aspect, the present invention concerns, a method for optimizing therapeutic efficacy of a tumor treatment with a molecule of the taxoid family, wherein the method comprises taking a cancer sample from a patient, determining the expression level of SHISA3 in said sample, comparing the expression level of SHISA3 to a reference expression level, wherein the under-expression of SHISA3 is predictive that a treatment with a molecule of the taxoid family in combination with a compound inhibiting the TGF-β signaling pathway is indicated for said patient and administering to the patients with under-expression of SHISA3 a treatment comprising a molecule of the taxoid family in combination with a compound inhibiting the TGF-β signalling pathway.

In yet another particular aspect, the present invention concerns a method for providing data useful for selecting a patient affected with a tumor for a treatment with a molecule of the taxoid family in combination with a compound inhibiting the TGF-β signaling pathway or determining whether a patient affected with a tumor is susceptible to benefit from a treatment with a molecule of the taxoid family in combination with a compound inhibiting the TGF-β signaling pathway, wherein the method comprises providing a cancer sample from said patient, determining the expression level of SHISA3 in said sample, comparing the expression level of SHISA3 to a reference expression level, wherein the under-expression of SHISA3 is predictive that a treatment with a molecule of the taxoid family in combination with a compound inhibiting the TGF-β signaling pathway is indicated for said patient and selecting patients with under-expression of SHISA3 for a treatment with a molecule of the taxoid family in combination with a compound inhibiting the TGF-β signalling pathway.

In a particular aspect, the present invention concerns a method for selecting a patient affected with a tumor for a treatment with a molecule of the taxoid family in combination with a compound inhibiting the TGF-β signaling pathway or determining whether a patient affected with a tumor is susceptible to benefit from a treatment with a molecule of the taxoid family in combination with a compound inhibiting the TGF-β signaling pathway, wherein the method comprises determining the expression level of SHISA3 in a cancer sample from said patient, comparing the expression level of SHISA3 to a reference expression level and selecting patients with under-expression of SHISA3 for a treatment with a molecule of the taxoid family in combination with a compound inhibiting the TGF-β signalling pathway. Optionally, the method further comprises a previous step of providing a cancer sample from said patient

In another particular aspect, the present invention concerns a method for selecting a patient affected with a prostate tumor for a treatment with a compound inhibiting the androgen axis in combination with a compound inhibiting the TGF-β signaling pathway or determining whether a patient affected with a prostate tumor is susceptible to benefit from a treatment with a compound inhibiting the androgen axis in combination with a compound inhibiting the TGF-β signaling pathway, wherein the method comprises providing a cancer sample from said patient, determining the expression level of SHISA3 in said sample, comparing the expression level of SHISA3 to a reference expression level, wherein the under-expression of SHISA3 is predictive that a treatment with a compound inhibiting the androgen axis in combination with a compound inhibiting the TGF-β signaling pathway is indicated for said patient and selecting patients with under-expression of SHISA3 for a treatment with a compound inhibiting the androgen axis in combination with a compound inhibiting the TGF-β signalling pathway.

The compound inhibiting the androgen axis can be selected from the group comprising the luteinising hormone releasing hormone (LHRH) agonists and androgen receptor antagonists.

All the embodiments disclosed above are also contemplated in the products, methods, kits and uses below.

The present invention also concerns, in a second aspect, a compound inhibiting the TGF-β signalling pathway for use in combination with a molecule of the taxoid family in the treatment of a cancer in which SHISA3 is under-expressed in comparison to a reference expression level, in particular a cancer that is resistant to a molecule of the taxoid family.

The compound inhibiting the TGF-β signalling pathway may be administered by any conventional route of administration such as oral, parenteral (including subcutaneous, intramuscular, intravenous and intradermal) or intratumoral (or in the vicinity of the tumor). The compound inhibiting the TGF-β signalling pathway may be administered as a single dose or in multiple doses. The amount of compound inhibiting the TGF-β signalling pathway to be administered has to be determined by standard procedure well known by those of ordinary skill in the art. Physiological data of the patient (e.g. age, size, and weight) and the routes of administration have to be taken into account to determine the appropriate dosage, so as a therapeutically effective amount will be administered to the patient.

The molecule of the taxoid family may be administered by any conventional route of administration such as oral, parenteral (including subcutaneous, intramuscular, intravenous and intradermal) or intratumoral (or in the vicinity of the tumor). The molecule of the taxoid family may be administered as a single dose or in multiple doses. The amount of molecule of the taxoid family to be administered has to be determined by standard procedure well known by those of ordinary skill in the art. Physiological data of the patient (e.g. age, size, and weight) and the routes of administration have to be taken into account to determine the appropriate dosage, so as a therapeutically effective amount will be administered to the patient.

The compound inhibiting the TGF-β signalling pathway and the molecule of the taxoid family may be administered simultaneously, separately or consecutively.

The present invention also concerns a product comprising a compound inhibiting the TGF-β signalling pathway and a molecule of the taxoid family, as a combined preparation for simultaneous, separate or sequential use, in particular for treating a cancer.

Preferably, the cancer to be treated is a cancer in which SHISA3 is under-expressed in comparison to a reference expression level. More preferably, the cancer is further resistant to a molecule of the taxoid family. In a particular embodiment, the molecule of the taxoid family is docetaxel.

The pharmaceutical composition is formulated in accordance with standard pharmaceutical practice (see, e.g., Remington: The Science and Practice of Pharmacy (20th ed.), ed. A. R. Gennaro, Lippincott Williams & Wilkins, 2000 and Encyclopedia of Pharmaceutical Technology, eds. J. Swarbrick and J. C. Boylan, 1988-1999, Marcel Dekker, New York) known by a person skilled in the art. Possible pharmaceutical compositions include those suitable for oral, rectal, topical (including transdermal, buccal and sublingual), or parenteral (including subcutaneous, intramuscular, intravenous, intratumoral and intradermal) administration. For these formulations, conventional excipient can be used according to techniques well known by those skilled in the art.

As described above, the patient is preferably a human, including adult, child and human at the prenatal stage. The terms "molecule of the taxoid family", "SHISA3" and "compound inhibiting the TGF-β signalling pathway"" are as defined above. The expression level of SHISA3 may be determined by measuring the quantity of SHISA3 protein or SHISA3 mRNA, as disclosed above, to be compared with a reference expression level. The reference expression level can be the expression level of SHISA3 in cell lines or tumors which are known to be sensitive to a treatment by the molecule of the taxoid family, as described above. Preferably, the reference expression level is the expression level of SHISA3 in a cancer cell line providing from a cancer of the same type than the cancer to be treated. Alternatively, the reference expression level is the expression level of SHISA3 in healthy cells of the patient. SHISA3 is considered as under-expressed if the expression level in the tumor of the patient is, after normalization, at least 20, 30, 40, 50, 60, 70, 80 or 90 % lower than the reference expression level, more preferably at least 50 % lower than the reference expression level. In a particular embodiment, the patient is affected with a prostate cancer and the molecule of the taxoid family is docetaxel.

In another aspect, the present invention concerns the use of a compound inhibiting the TGF-β signalling pathway in combination with a molecule of the taxoid family for the manufacture of a medicament for treating cancer. Preferably, the cancer to be treated is a cancer in which SHISA3 is under-expressed in comparison to a reference expression level. More preferably, the cancer is further resistant to a molecule of the taxoid family. In a particular embodiment, the molecule of the taxoid family is docetaxel.

The present invention also concerns the use of a compound inhibiting the TGF-β signalling pathway for the manufacture of a medicament for treating cancer in which SHISA3 is under-expressed in comparison to a reference expression level. More preferably, the cancer is further resistant to a molecule of the taxoid family. In a particular embodiment, the molecule of the taxoid family is docetaxel.

The present invention further concerns a method for treating a cancer in a patient, wherein the method comprises the step of administering a therapeutically effective amount of a combination of a molecule of the taxoid family and a compound inhibiting the TGF-β signalling pathway to a subject having a tumor in which SHISA3 is under-expressed in comparison to a reference expression level. Optionally, the cancer is further resistant to a molecule of the taxoid family. The compound inhibiting the TGF-β signalling pathway and the molecule of the taxoid family may be administered simultaneously, separately or consecutively.

In a third aspect, the present invention concerns a method for screening or identifying a compound suitable for improving the treatment of a cancer with a molecule of the taxoid family or for reducing the resistance development during the treatment of a cancer with a molecule of the taxoid family.

In a first embodiment, the method comprises providing a cell-line, preferably a cell line in which SHISA3 is under-expressed in comparison to a reference expression level; contacting said cell-line with a test compound, determining the activity level of the TGF-β signalling pathway; and selecting the compound which inhibits the TGF-β signalling pathway. The cell line used in this screening method is preferably a cell line in which SHISA3 is under-expressed in comparison to a reference expression level. Such cell line may be identified by determining the expression level of SHISA3 in said cell line by measuring the quantity of SHISA3 protein or SHISA3 mRNA, as disclosed above, and comparing said expression level with a reference value. The cell line used to determine the reference expression level of SHISA3 may be chosen as described above. Preferably, the cell line used to determine the reference expression level is a prostate cancer cell line sensitive to a molecule of the taxoid family, and more preferably a prostate cancer cell line sensitive to docetaxel. SHISA3 is under-expressed in a cell line if its expression level, after normalization, is at least 20, 30, 40, 50, 60, 70, 80 or 90 % lower than the reference expression level. Preferably, the expression level of SHISA3 is at least 50 % lower than the reference expression level.

In a particular embodiment, the cell line in which SHISA3 is under-expressed is a cancer cell line resistant to a molecule of the taxoid family, preferably a prostate cancer cell line resistant to a molecule of the taxoid family, and more preferably a docetaxel-resistant prostate cancer cell line. The methods for preparing a cell line resistant to a molecule of the taxoid family are well-known by the artisan skilled in the art. In particular, a protocol is disclosed in Patterson et al. (Oncogene. 2006, 25(45), pp. 6113-22). The cells can be contacted with a first dose of the cytotoxic drug. After the clones sensitive to the first dose are no longer present, the surviving cells repopulate the flask and continue to divide through about 4 passages. Then, they are incubated with a higher second dose of the cytotoxic drug. The same methodology is followed with each increase in the concentration of the cytotoxic drug until to reach the dose of the cytotoxic drug at which the cells have to be resistant. In a particular embodiment, the cell line in which SHISA3 is under-expressed is selected from the group consisting of the cell lines resistant to a molecule of the taxoid family obtained from the prostate cancer cell lines IGR-CaP1, PC3, LNCaP, VCaP and 22RV1. Preferably, the cell line in which SHISA3 is under-expressed is selected from the group consisting of docetaxel-resistant cell lines obtained from the cell lines IGR-CaP1, PC3, LNCaP, VCaP and 22RV1. More preferably, the cell line in which SHISA3 is under-expressed is a cell line resistant to at least 0.5 nM of docetaxel and obtained from the cell line IGR-CaP1, PC3, LNCaP, VCaP and 22RV1. PC3, LNCaP, VCaP and 22RV1 cell lines are commercially available (ATCC, catalogue numbers: CRL-1435, CRL-1740, CRL-287, and CRL-2505 respectively) The cell line IGR-CaP1 has been described in the patent application WO2010119001 and has been deposited at the CNCM (Collection Nationale de Culture de Microorganismes at the Pasteur Institute, 25 rue du Dr. Roux, F-75724 Paris Cedex 15, France) on February 10, 2009 under number 1-4126. In a preferred embodiment, the cell line in which SHISA3 is under-expressed is the prostate cancer cell line IGR-CaP1-R100 which is resistant to 100 nM of docetaxel, or a progeny thereof. This cell line has been described in the patent application PCT/EP2010/054739 and has been deposited at the CNCM (Collection Nationale de Culture de Microorganismes at the Pasteur Institute, 25 rue du Dr. Roux, F-75724 Paris Cedex 15, France) on February 10, 2009 under number 1-4127.

In another embodiment, the method for screening or identifying a compound suitable for improving the treatment of a cancer with a molecule of the taxoid family or for reducing the resistance development during the treatment of a cancer with a molecule of the taxoid family, comprises providing a cell-line sensitive to the molecule of the taxoid family, contacting said cell-line with a test compound and the molecule of the taxoid family, determining the expression level of SHISA3 and selecting the compound which inhibits the appearance of an under-expression of SHISA3 or which inhibits the appearance of resistant cells. The cell line sensitive to the molecule of the taxoid family may be easily chosen by the skilled person in the art. For instance, this cell line may be selected from the group consisting of IGR-CaP1, PC3 and LNCaP cell lines. Preferably, the cell line sensitive to a molecule of the taxoid family is sensitive to docetaxel. More preferably, this cell line cannot survive in a medium containing 1 nM of docetaxel. The sensitive cell line is contacted with a test compound and a molecule of the taxoid family. Preferably the molecule of the taxoid family is docetaxel. In a particular embodiment, the sensitive cell line is cultured *in vitro* in a suitable medium well known by the skilled person, such as RPMI medium, complemented with a molecule of the taxoid family and the test compound. Cells are then incubated in suitable conditions, e.g. 37°C with 5% CO2. In one embodiment, the test compound is selected if it inhibits the appearance of an under-expression of SHISA3. The expression level of SHISA3 may be monitored by measuring the quantity of SHISA3 protein or SHISA3 mRNA, as disclosed above. Preferably, the test compound is selected if the expression level of SHISA3 does not decrease of more than 20 % during the culture, more preferably of more than 10 %. In another embodiment, the test compound is selected if it inhibits the appearance of cells resistant to the molecule of the taxoid family. The number of resistant cells in culture comprising the test compound and the taxoid is compared to the number of resistant cells in culture with said taxoid but without said test compound. The test compound is selected if it allows to decrease the number of cells resistant to the molecule of the taxoid family which appear during the culture. Preferably, the test compound is selected if it inhibits the appearance of cells resistant to the molecule of the taxoid family.

In a fourth aspect, the present invention concerns a kit for
- selecting a patient affected with a tumor for a treatment with a molecule of the taxoid family in combination with a compound inhibiting the TGF-β signalling pathway; and/or
- screening or identifying a compound suitable for improving the treatment of a cancer with a molecule of the taxoid family,
wherein the kit comprises detection means selected from the group consisting of a pair of primers, a probe and an antibody specific to SHISA3, and a combination thereof.

In a particular embodiment, the kit comprises at least a pair of primers and a probe specific to SHISA3. Preferably, the probe specific to SHISA3 is a Taqman probe. In another particular embodiment, the kit comprises at least an antibody specific to SHISA3 and a probe or a pair of primers specific to SHISA3.

The present invention also concerns, in a fifth aspect, the use of the expression level of SHISA3 as a marker for selecting a patient affected with a tumor for a treatment with a molecule of the taxoid family in combination with a compound inhibiting the TGF-β signalling pathway. The expression level of SHISA3 may be assessed as described above.

In a sixth aspect, the present invention finally concerns, the use of a kit for selecting a patient affected with a tumor for a treatment with a molecule of the taxoid family in combination with a compound inhibiting the TGF-β signaling pathway and/or screening or identifying a compound suitable for improving the treatment of a cancer with a molecule of the taxoid family, wherein the kit comprises detection means selected from the group consisting of a pair of primers, a probe and an antibody specific to SHISA3, and a combination thereof.

In a particular embodiment, the kit comprises at least a pair of primers and a probe specific to SHISA3. Preferably, the probe specific to SHISA3 is a Taqman probe. In another particular embodiment, the kit comprises at least an antibody specific to SHISA3 and a probe or a pair of primers specific to SHISA3.

Further aspects and advantages of the present invention will be described in the following examples, which should be regarded as illustrative and not limiting its scope.

### EXAMPLES

### Materials and Methods

### Cell culture and selection of docetaxel-resistant clones

The parental and docetaxel-resistant IGR-CaP1 (Chauchereau A et al, Exp Cell Res., 2011, 317(3), pp. 262-75) and PC3 human prostate cancer cell lines were maintained in RPMI1640 medium supplemented with 10% FBS. The parental PC3 cell line and human embryonic kidney HEK-293 cells were purchased from ATCC. docetaxel-resistant PC3 clones were selected by exposing cells to docetaxel in a dose-escalation manner as described for IGR-CaP1 (Nakouzi NA et al, Oncotarget, 2014, 5(3), pp.667-78). Both resistant cell lines were treated monthly with the maximal dose of docetaxel to maintain the resistant phenotype (100nM or 12nM for IGR-CaP1-R and PC3-R cells, respectively). HEK cells were maintained in DMEM medium supplemented with 10% FBS.

### Plasmids and reagents

The nucleotide sequence corresponding to the human *SHISA3* cDNA with an in-frame insertion of the Flag tag sequence at position 259 was purchased from Genewiz. The *SHISA3-Flag* coding sequence was inserted by standard cloning into the pLv-2028 provided by D. Compagno (IQUIBICEN-CONICET, Universidad de Buenos Aires, Argentina). The pLv-*SHISA3-Flag* vector expresses GFP as reporter under the control of eukaryotic EF-1a promoter. The expression vector for HA-TGFβR2 (Wrana JL et al, Cell, 1992, 71(6), pp. 1003-14.) was provided by N. Théret (Inserm U620, Rennes, France). TGFβ1 was purchased from R&D Systems and was resuspended in 4mM HCl supplemented with 1mg/ml bovine serum albumin. LY2157299 inhibitor was obtained from AbMole Biosciences and was resuspended in DMSO.

### Quantitative real-time RT-PCR

The TissueScan Human Normal and TissueScan Cancer cDNA Arrays (Origene) were used to provide template cDNA for 48 major human tissues and for 19 specific cancers. TaqMan gene expression assays were used for human *SHISA3* gene (Hs01380806_ml), human SNAI1/Snail (Hs00195591_ml) and human ZEB1 (Hs00232783_ml) with GAPDH as endogenous control. Total RNA was extracted using Trizol from parental and docetaxel-resistant cells as previously described (Nakouzi NA et al, Oncotarget, 2014, 5(3), pp.667-78). Real-time quantification was performed using TaqMan gene expression master mix as described (Chauchereau A et al, Exp Cell Res., 2011, 317(3), pp. 262-75) on Viia7 System (Applied Biosystems). The ΔΔCT method was used to quantify transcripts.

### Microarray Assay

Gene expression was profiled using a 4×44K Human Whole Genome (AMADID 14850) expression array (Agilent Technologies, Santa Clara, Cal.) as previously described (Nakouzi NA et al, Oncotarget, 2014, 5(3), pp.667-78). Microarray images were analysed by using Feature Extraction software version (10.7.3.1) from Agilent technologies. Defaults settings were used.

### Microarray data processing and analysis

Raw data files from Feature Extraction were imported into R with LIMMA, an R package from the Bioconductor project, and processed as follow: gMedianSignal and rMedianSignal data were imported, controls probes were systematically removed, and flagged probes (gIsSaturated, gIsFeatpopnOL, gIsFeatNonUnifOL, rIsSaturated, rIsFeatpopnOL, rIsFeatNonUnifOL) were set to NA. Intra-array normalization was performed by loess normalization, followed by a quantile normalization of both Cy3 and Cy5 channel. Then inter-array normalization was performed by quantile normalization on M values. To get a single value for each transcript, taking the mean of each replicated probes summarized data. Missing values were inferred using KNN algorithm from the package 'impute' from R bioconductor.

Normalized data were then analyzed. To assess differentially expressed genes between two groups, we start by fitting a linear model to the data. Then we used an empirical Bayes method to moderate the standard errors of the estimated log-fold changes. The top-ranked genes were selected with the following criteria: an absolute fold-change > 2 and an adjusted p-value (FDR) < 0.05.

### MicroRNA extraction and expression profiling

MicroRNAs were reverse transcribed to cDNA using the Taqman Megaplex Primer Pools A kit (Applied Biosystems). RT-qPCR was performed using TaqMan Array Human Micro RNA A Cards (Applied Biosystems) as described by the supplier and quantified on 7900HT system (Applied Biosystems). Independent biological replicates were used for each cell lines. The relative quantification value of the target, normalized to a control, was calculated by the comparative Ct methods. MicroRNAs expressed at a Ct value over 32 in both parental and resistant cell lines were eliminated from the analysis. RNU6B and endogenous hsa-miR-24 were used as internal controls. Fold changes represent the mean of the fold change values obtained with the two controls. Using this procedure, MiR signatures were extracted for each cell line with a fold change cut-off set at 1.5 (Table SII).

### EMT induction

2,5.10⁴ IGR-CaP1 cells and 5.10⁴ PC3 cells were plated in 6-well plates for 24 hours then transiently transfected with 10nM synthetic siRNA targeting SHISA3 gene (Stealth RNAi™, siSHISA3-94 : HSS152694, siSHISA3-95 : HSS152695, and siSHISA3-96 : HSS152696) using lipofectamine RNAiMax (Invitrogen) or treated with 10ng/ml TGFβ1. After 36 hours, a second treatment was performed in order to maintain EMT induction over 5 days. Negative controls were obtained by treating cells with siRNA negative controls (siNT) (Invitrogen) or TGFβ1 vehicle. Whole cell extracts were prepared in RIPA buffer complemented with proteases inhibitors (Roche) and phosphatase inhibitors (Sigma).

### Western Blot analysis

Fifty µg of lysates were used for Western-Blot probed with specific antibodies: SHISA3 (Abgent), E-cadherin (BD Biosciences, clone 36), N-Cadherin (BD Biosciences, Clone 32), β-catenin (Santa Cruz, clone 12F7), Vimentin (BD Pharmingen, clone RV202), Occludin (Life Technologies, Clone OC-3F10), Plakoglobin (Sigma-Aldrich, Clone 15F11), Snail (Cell Signaling, Clone C15D3), ZEB1 (Cell Signaling, D80D), EGFR (Cell Signaling, Clone D38B1), TGFβR2 (Santa Cruz, Clone E-6), Ubiquitin (Santa Cruz, Clone P4D1), Flag (Sigma, M2). β-actin (Sigma-Aldrich, Clone AC-15) was used as a loading control. Immunoblot analyses were performed using the enhanced chemoluminescence-based detection kit (Pierce).

### Wound healing assay

IGR-CaP1 and PC3 cells were plated in 6-well plates then EMT was induced as described above. Wounds were realized after 5 days of treatment, and then the medium was replaced for medium without FBS. Images were taken at starting point, 12 hours and 24 hours after the wound using an inverse microscope (EVOS AMEX1200, Life Technologies). Area between the wound edges is quantified with the ImageJ software (NIH).

### Immunoprecipitation and proteomic analysis

For immunoprecipitation, IGR-CaP1, PC3 and HEK cells were transfected with the pLV-*SHISA3-Flag* lentiviral expression vector or empty vector using jetPRIME transfection reagent (Ozyme). 48h after transfection, cell extracts were prepared using cryolysis. Cells were trypsinised and resuspended in 20mM HEPES pH 7.5, 1.2% polyvinilpirrolidone and protease inhibitors. Cellular suspension was added to liquid nitrogen, and mechanically disrupted using the Tissue Lyser system (Qiagen). Protein extracts were obtained by incubating the cryopowder with a solution of 120mM NaCl, 20mM HEPES, 1mM EDTA, 5% glycerol, 1% Briij97, protease inhibitors, at 4°C. SHISA3, EGFR or TGFβR2 proteins were co-immunoprecipitated using standard procedures and subjected to immunoblotting, in order to verify the efficiency of the immunoprecipitation procedure. The proteins eluted with FLAG peptide were digested with trypsin, using the FASP protocol (Wiśniewski JR et al, Nat Methods, 2009, 6(5), pp. 359-62), and the resulting peptides were desalted and concentrated using Vivapure C-18 Micro Spin Columns (VS-RP218L24_Sartorius stedim). The analyses of peptide mixtures were performed on EASY 1000nLC Q-EXACTIVE (Thermo Scientific), using EASY-Spray nanocolumn (ES800 15cm 75um), 300 nanoliter/min flow and 2h gradient of acetonitrile (starting concentration 5% Acetonitrile and final 35%). The mass resolution for MS was set at 70000, whereas the mass resolution for MS/MS was set for 17500, and the collision energy for set for 27. The acquired data were analysed with Proteome Discoverer Software package using Mascot search engine.

### Clonogenicity assay

IGR-CaP1 and IGR-CaP1-R cells were plated in 6 well plates for 24 hours. Cells were pre-treated with 25µM LY2157299 or vehicle for 72 hours, and then co-treated with LY2157299 and docetaxel (12nM for parental cells, 100nM for resistant cells). After 2 weeks, clones were methanol fixed and stained using Crystal violet (sigma). Clones were counted using ImageJ software.

### Flow cytometry

Flow cytometry was used to quantify apoptosis in LY2157299 and/or docetaxel treated cells. Cells were stained using BD Pharmingen™ FITC Annexin V Apoptosis Detection Kit (BD Biosciences, 556547) according to the supplier instructions. Fluorescence was measured with FACSCalibur system (Becton Dickinson).

### Immunofluorescence microscopy

Cells were plated on coverslips and grown for 2 days. Depending on experimental procedures, cells were transiently transfected for 48h with the lenti*-Flag-SHISA3* expression vector lentiviral or empty vector using jetPRIME transfection reagent (Ozyme). Cells were fixed in 4% formaldehyde and permeabilized with 0.2% triton X100. Cells were labelled with specific antibodies followed by incubation with dye-conjugated antibodies (Molecular Probes). Endoplasmic reticulum and Golgi apparatus stainings were obtained by using Calnexin (Santa Cruz Biotechnology) and GM130 (BD Biosciences) antibodies respectively. Images were acquired on confocal microscope (Leica TCS SPE) or fluorescence microscope (Zeiss Axioplan 2). Tridimensional reconstructions of stacked confocal images were obtained using Imaris software (Bitplane).

### In vivo docetaxel-resistant prostate cancer model

1.10⁶ IGR-CaP1 cells and 1.10⁷ IGR-CaP1-R25 cells have been injected in nude athymic mice (NC-nu/nu). Xenografted mice received successive treatments of docetaxel or vehicle when the size of the tumors reached 100mm3.

### Results

### Identification of SHISA3 as the most under-expressed gene in docetaxel-resistant cells

To investigate the molecular mechanisms implicated in resistance to taxane, the inventors compared gene expression profiles of docetaxel-resistant versus parental cells. A previous analysis obtained in the IGR-CaP1 prostate cancer cells had showed that *SHISA3* was the most under-expressed gene in the resistant cells (Fold-change = -187, p-value <5x10⁻²) (Nakouzi NA et al, Oncotarget, 2014, 5(3), pp. 667-78). The inventors generated a second model of docetaxel-resistant cells by exposing PC3 cells to increasing docetaxel concentration in long term cultures. A signature of 420 genes potentially implicated in resistance in PC3 models was obtained (data not shown) and *SHISA3* gene was also down-regulated in docetaxel-resistant PC3-R12 cells (Fold-Change=-2.53). Extinction of the *SHISA3* gene was confirmed by RT-qPCR in both resistant cell lines (Figure 1A). The *SHISA3* gene encodes a protein of 26 kDa which was detected in western blot in parental IGR-CaP1 cells and to a lesser extent in PC3 cells, but was undetectable in docetaxel-resistant IGR-CaP1 and PC3 cell lines (Figure 1B).

To date the *SHISA3* gene has been poorly described in humans; the expression of *SHISA3* in various cell lines was essentially determined by RT-PCR. Various expression levels were observed in prostate cancer cell lines, the IGR-CaP1 and PC3 cell lines showing the highest *SHISA3* expression level. *SHISA3* was also detected in HEK kidney cells and in the SKMEL-31 melanoma cells (Figure 1C). In addition, *SHISA3* expression was determined by quantitative RT-PCR in a panel of normal tissues showing that *SHISA3* was preferentially expressed in genitourinary tissues and in the digestive system (Figure 1D). Interestingly, *SHISA3* expression is higher in normal prostate tissue than in tumors prostate cells where *SHISA3* expression is generally lost. This differential expression was also significantly observed in renal, colon and lung tumors (Figure IE).

According to previous studies in Xenopus and in mouse, the inventors were expecting to observe SHISA3 in the endoplasmic reticulum (ER) (Yamamoto A et al, Cell, 2005, 120(2), pp. 223-35; Nagano T et al, Dev Camb Engl, 2006, 133(23), pp. 4643-54; Furushima K et al, Dev Biol, 2007, 306(2), pp. 480-92). Surprisingly, confocal microscopy analysis of SHISA3 subcellular localization in HEK cells over-expressing Flag-tagged SHISA3 showed that SHISA3 was localized both in a large cytoplasmic structure and in discontinuous speckles at the cytoplasmic membrane (Figure IF). The same cytoplasmic localization was also observed in PC3 transfected cells (data not shown), without any overlapping with the ER labeling but overlapped with the Golgi apparatus.

Insofar SHISA3 loss has only been observed in the *in vitro* resistant models. The inventors have also quantified SHISA3 gene expression in animal models. SHISA3 expression was measured by quantitative PCR in sensitive tumor (IGR-CAP/megix) and in resistant tumors (IGR-CAP1-R25). Tumors derived from IGR-CAP1/megix cell line treated with the vehicle have a high expression of SHISA3 gene (Figure 1G). It is also observed that treatment with docetaxel in this model induces a strong decrease in the expression of SHISA3. On the opposite, in tumors derived from the IGR-CAP1-R25 cell line, SHISA3 is very little expressed and docetaxel treatment did not affect its expression.

All together, the inventors showed that SHISA3 is highly down-regulated in the chemoresistant cell lines, suggesting a role of this protein in the mechanism of docetaxel-resistance in prostate cancer.

### SHISA3 down-regulation in docetaxel-resistant cells is correlated with EMT

To determine, the pathways implicated in docetaxel resistance, the inventors first analyze a miRNA signature which was differentially expressed between docetaxel-resistant and parental cells in the two cell lines IGR-CaP1 and PC3. Quantification of miRNA expression levels by RT-qPCR led to the identification of a number of miRNAs that have been implicated in EMT (Figure 2A). Among the repressed miRNAs, many have been reported previously as critical regulators of EMT, such as the miR-200 family (miR-200b, miR-200c, miR-141) and miR-203 (16,32). The comparison of transcriptional gene signatures of docetaxel-resistant IGR-CaP1 and PC3 cells with the EMT gene signature (Gröger CJ et al, PloS One, 2012, 7(12), p. e51136), showed that EMT pathway genes were significantly enriched in both the IGR-CaP1-R signature (9.25%; p-value=4.10x10⁻¹⁵) and in the PC3-R signature (11%; p-value=2.12x10⁻²²) (Fisher test) (Figure 2B). Thus, these results suggest that EMT is a major process in docetaxel resistance in these models.

Indeed, docetaxel-resistant cells do not have the same morphological characteristics as the parental cell lines (Figure 2C). PC3-R cells, when individualized, showed a fibroblast-like phenotype, whereas the resistant IGR-CaP1-R cells showed an increase in cell size, suggesting changes in the intercellular junctions, typical of mesenchymal cells. Therefore, the inventors studied the expression of known epithelial and mesenchymal markers by western-blotting and immunofluorescence.

Western blot analysis showed the loss of epithelial markers such as E-cadherin (CDH1) and β-catenin (CTNNB1) in both docetaxel-resistant cells concomitant with a gain of expression of mesenchymal markers such as N-cadherin (CDH2) and Vimentin (VIM) (Figure 2D). Immunofluorescence staining confirmed the loss of plasma membrane E-Cadherin in resistant cells (Figure 2D).

The change of cellular morphology of docetaxel-resistant cells suggests a disorganization of cellular junctions. Intercellular junctions are composed of tight and adherent junctions including desmosomes, characterized by the expression of various proteins such as claudins, occludins, Zona Occludens (ZO) proteins, plakoglobin or desmoglein. To study the junctions, the inventors looked at the expression of the tight-junction protein occludin (OCLN) and plakoglobin (JUP). Western blot and immunofluorescence analysis in Figure 2E and 2F showed the decrease of OCLN and JUP in resistant cells of both cell lines respectively.

The reorganization of the cytoskeletal architecture depends on the expression of master regulators including Snail (SNAI1), Slug (SNAI2), TWIST and Zinc-finger E-box binding (ZEB) transcription factors, which have been characterized in EMT. Figure 2G shows that ZEB1 was overexpressed in both docetaxel-resistant cells, whereas SNAI1 was increased only in IGR-CaP1-R by qRT-PCR. The results were confirmed by Western-blotting. Immunofluorescence shows the nuclear localization of ZEB1 in resistant cells.

Overall, these results showed that docetaxel-resistant cells have undergone a profound remodeling and have become mesenchymal cells.

### SHISA3 silencing promotes EMT

To question the functional relationship between the repressed *SHISA3* gene and EMT in docetaxel-resistant cells, the inventors investigate if the resistant phenotype was mimicked in both parental cell lines by using siRNA against *SHISA3* (siSHISA3).

Loss of tight junctions is the first event in the process of EMT. Silencing of SHISA3 induced the loss of OCLN in both cell lines, suggesting a link between loss of tight junctions and down-regulation of SHISA3 in resistant cells (Figure 3A).

The inventors then wondered if this loss was inducing morphological changes, as observed in resistant cells. Strikingly, looking at cells in phase contrast microcopy, SHISA3 knockdown induced morphological changes in both IGR-CaP1 and PC3 cell lines (the major effect was observed with siSHISA3-3, Figure 3B).

OCLN results were confirmed with a complementary analyze of epithelial and mesenchyme markers expression in SHISA3 knocked down cells. As observed with docetaxel-resistant cells, siSHISA3 induced a decrease of the epithelial marker CDH1 and an increase in mesenchymal proteins CDH2 and Snail/SNAI1 (Figure 3C) suggesting that *SHISA3* down-regulation induces EMT.

As EMT is often accompanied by an increased cell migration, the inventors measured cell migration by performing a wound healing assay 5 days after siSHISA3 transfections (Figure 3D). Consistently, silencing of SHISA3 significantly increased cell migration after 24h in both cell types.

Together, these results show a functional relationship between SHISA3 and EMT suggesting the importance of this gene in the early steps of EMT.

### SHISA3 interacts with TGFβR2

SHISA3 homologues were originally described as inhibitors of Wnt and FGF signaling pathways. Based on sequence similarity searches upon the SHISA gene family, Pei *et al.* proposed that SHISA3 may correspond to transmembrane adapters that could interact with ubiquitin ligases to regulate membrane proteins such as cell surface receptors (Pei J et al, Cell Signal, 2012, 24(3), pp. 758-69).
To identify cytosolic proteins potentially interacting with SHISA3, a comprehensive proteomic profiling of co-immunoprecipitated proteins was performed. First, a SHISA3 expression vector containing an internal Flag Tag to let the cytoplasmic moiety of the protein free to interact with cytoplasmic proteins was constructed (Figure 4A). Flag-tagged *SHISA3* was transiently expressed in IGR-CaP1 and PC3 cells, and Flag co-precipitated proteins were analyzed by nano-MS/MS (Orbitrap Q-Exactive) after tryptic digestion. Using this approach, protein extraction with milder detergent Brij 97 allows to retain both direct and indirect interactions. Twenty-eight proteins from a total of 3741 were specifically identified in both cell lines in the presence of *SHISA3* expressing vector (data not shown). By clustering these proteins using cancer networks analysis IngenuityⒸ software, the inventors identified one significant network which highlighted the EGFR and the TGBβR2 signaling pathways (data not shown), suggesting that SHISA3 may be implicated in EGFR and TGFβR2 signaling.

To test this hypothesis, cells overexpressing SHISA3-Flag were immunoprecipitated either with antibodies against TGFβR2, EGFR, and Flag-Tag or with non-specific immunoglobulines. Co-immunoprecipations from IGR-CaP1 cells showed that SHISA3 interact specifically with TGFβR2 whether the immunoprecipitating antibody was anti-TGFβR2 or anti-SHISA3 (anti-Flag) (Figure 4B). EGFR did not co-precipitate with SHISA3. Identical results were observed in PC3 and in HEK cells (Figure 4B). Endogenous SHISA3 was also co-immunoprecipitated with TGFβR2 in IGR-CaP1 and in PC3 cells (Figure 7). To confirm this interaction, the inventors examined the subcellular localization of both SHISA3 and TGFβR2 by confocal microscopy after ectopic expression of *HA-TGFβR2* and *SHISA3-Flag* in HEK cells. SHISA3 was preferentially observed in both speckles near plasma membrane and in cytoplasmic granules where it co-localized with TGFβR2 (Figure 4C). As SHISA3 was observed in cytoplasmic Golgi, it is hypothesized that the complex SHISA3/TGFβR2 is localized in Golgi. Indeed, TGFβR2 has been reported to localize in Golgi apparatus (Wells RGet al, J Biol Chem, 1997, 272(17), pp. 11444-51).

To determine if SHISA3 was involved in the regulation of TGFβ signaling through its interaction with TGFβR2, parental IGR-CaP1 and PC3 cells were treated with TGFβ for 5 days and then, the effect on the expression of SHISA3 and EMT markers was analyzed (Figure 4D). A decrease in SHISA3 expression was observed in the treated cells compared to control cells. A decrease of CDH1 expression and increase in CDH2 and VIM expression were also observed in treated cells. Therefore, TGFβ mimic the effect triggered by loss of SHISA3 in resistant cells and SHISA3 knockdown in parental cells.

Together, these results suggest that in epithelial cells, SHISA3 interacts with TGFβR2 receptor in order to inhibit TFGβ signaling. In resistant cells, absence of SHISA3 alleviates this inhibition thus rendering TFGβR2 available to interact with TGFβ in order to maintain the mesenchymal state.

### SHISA3 regulates TGFβR2 through ubiquitination

SHISA3 was proposed to inhibit transmembrane receptors by interacting with ubiquitin ligase (Pei J et al, Cell Signal, 2012, 24(3), pp.758-69). Herein, poly-ubiquitinated forms of TGFβR2 should be observed in the presence of SHISA3. To test this hypothesis, immunoprecipitation was performed with TGFβR2 antibody in parental and resistant IGR-CaP1 cells. Immunoprecipitated proteins were subjected to an immunoblot analysis and revealed with anti-TGFβR2 and anti-ubiquitin antibodies. Figure 5A showed that endogenous TGFβR2 was expressed as a high molecular weight receptor in these cells, corresponding to highly glycosylated forms of the receptor (Wells RG et al, J Biol Chem, 1997, 272(17), pp. 11444-51; Luga V et al, Biochem J, 2009, 421(1), pp. 119-31). This non-ubiquitinated form was expressed to the same extent in parental and resistant cells. Moreover, ubiquitinated forms of TGFβR2 were much more co-immunoprecipitated in the SHISA3-expressing parental cells. This result suggested that the interaction between SHISA3 and TGFβR2 triggers the ubiquitination of TGFβR2 which inhibits its signaling. To confirm this hypothesis, the impact of ectopically expressed SHISA3 on the ubiquitination of TGFβR2 in IGR-CaP1 cells was evaluated in the presence or not of co-expressed HA tagged-ubiquitin vectors (Figure 5B). The results show that the expression of SHISA3 highly increased the ubiquitinated forms of endogenous TGFβR2 (compare lane 2 and lane 4). These data showed that SHISA3 increases the ubiquitination of TGFβR2 suggesting that it participates in the regulation of TGFβR2 expression.

### Inhibition of TGFβR2 signaling increases cell death of docetaxel-resistant cells

The interaction between SHISA3 and TGFβR2 may prevent its interaction with TGFβR1 and impedes TGFβ signaling, maintaining cells in an epithelial state. In resistant cells, where SHISA3 is absent, TFGβR2 would on the opposite be able to trigger and to maintain the mesenchymal state. The inventors thus hypothesized that blocking TFGβR2 in resistant mesenchymal cells could restore their sensibility to docetaxel and induce, in combination with docetaxel, the death of resistant cells.

Inhibition of TGFβR1 is a strategy to inhibit TGFβ signaling. LY2157299 is a small molecule inhibitor of TGF-βRI which is the most advanced TGFβ signaling inhibitor under clinical development. Figure 6 shows that treatment of cells with 25µM of LY2157299 alone did not affect the survival of parental and resistant cells. However, when combined with 100nM of docetaxel, the survival of the resistant cells was significantly decreased (more than 50% of colonies). These results demonstrate that the targeting of the TGFβ signaling pathway with LY2157299 in combination with docetaxel leads to the death of docetaxel-resistant cells, thus reversing the resistance phenotype.

## Claims

1. An *in vitro* method for selecting a patient affected with a tumor for a treatment with a molecule of the taxoid family in combination with a compound inhibiting the TGF-β signalling pathway or for determining whether a patient affected with a tumor is susceptible to benefit from a treatment with a molecule of the taxoid family in combination with a compound inhibiting the TGF-β signalling pathway, wherein the method comprises:
(a) determining the expression level of SHISA3 in a cancer sample from said patient,
(b) comparing the expression level of SHISA3 to a reference expression level, wherein the under-expression of SHISA3 is predictive that a treatment with a molecule of the taxoid family in combination with a compound inhibiting the TGF-β signalling pathway is indicated for said patient and
(c) selecting patients with under-expression of SHISA3 as suitable for a treatment with a molecule of the taxoid family in combination with a compound inhibiting the TGF-β signalling pathway.

2. A compound inhibiting the TGF-β signalling pathway for use in combination with a molecule of the taxoid family in the treatment of a cancer in which SHISA3 is under-expressed in comparison to a reference expression level.

3. The method according to claim 1 or the compound according to claim 2, wherein said cancer is resistant to a molecule of the taxoid family.

4. The method according to claim 1 or 3 or the compound according to claim 2 or 3, wherein the expression level of SHISA3 is determined by measuring the quantity of SHISA3 protein or SHISA3 mRNA.

5. The method according to anyone of claims 1, 3 or 4 or the compound according to anyone of claims 2 to 4, wherein the reference expression level is the expression level of SHISA3 in cell lines or tumors sensitive to the treatment by the molecule of the taxoid family.

6. The method or the compound according to claim 5, wherein the cell line is IGR-CaP1.

7. The method according to any one of claims 1 or 3 to 6 or the compound according to any one of claims 2 to 6, wherein the molecule of the taxoid family is selected from the group consisting of paclitaxel (taxol®), docetaxel (taxotere®), larotaxel, cabazitaxel (XRP6258), BMS-184476, BMS-188797, BMS-275183, ortataxel, RPR 109881A, RPR 116258, NBT-287, PG-paclitaxel, ABRAXANE®, Tesetaxel, IDN 5390, Taxoprexin, DHA-paclitaxel, and MAC-321, Genexol-PM, LEP (LEP-ETU), Tocosol® paclitaxel, SB-T-1213, SB-T-1214, SB-T-1216, SB-T-1217, SB-T-121303, SB-T-121303021, SB-T-12130301, and a combination thereof.

8. The method according to any one of claims 1 or 3 to 7 or the compound according to any one of claims 2 to 7, wherein the molecule of the taxoid family is docetaxel.

9. The method according to any one of claims 1 or 3 to 8 or the compound according to any one of claims 2 to 8, wherein the cancer is selected from the group consisting of the prostate cancer, the lung cancer, the breast cancer, the gastric cancer, the kidney cancer, the ovarian cancer, the hepatocellular cancer, the osteosarcoma, the melanoma, the hypopharynx cancer, the oesophageal cancer, the endometrial cancer, the cervical cancer, the pancreatic cancer, the liver cancer, the brain cancer, the neuroendocrine tumors, the malignant tumor of the muscle, the adrenal cancer, the thyroid cancer, the uterine cancer, the skin cancer, the bladder cancer, and the head and neck cancer, preferably the prostate cancer.

10. The method according to any one of claims 1 or 3 to 9 or the compound according to any one of claims 2 to 9, wherein the compound inhibiting the TGF-β signaling pathway is an inhibitor of the TGF-β receptor, preferably an inhibitor of the TGF-β receptor I.

11. The method or the compound according to claim 10, wherein the inhibitor of the TGF-β receptor I can be selected in the group consisting of: LY2157299 (Galunisertib), SB431542, LY2109761, SB525334, SB505124, RepSox, GW788388, LY364947, A 83-01, SD 208, D 4476, TGF-β RI Kinase Inhibitor VIII, Ki268994, preferably, the inhibitor of the TGF-β receptor I is LY2157299.

12. Use of a kit for selecting a patient affected with a tumor for a treatment with a molecule of the taxoid family in combination with a compound inhibiting the TGF-β signaling pathway and/or screening or identifying a compound suitable for improving the treatment of a cancer with a molecule of the taxoid family, wherein the kit comprises detection means selected from the group consisting of a pair of primers, a probe and an antibody specific to SHISA3, and a combination thereof.

13. Use of the expression level of SHISA3 as a marker for selecting a patient affected with a tumor for a treatment with a molecule of the taxoid family in combination with a compound inhibiting the TGF-β signalling pathway.

## Patentansprüche

1. Ein in vitro Verfahren zur Auswahl eines mit einem Tumor befallenen Patienten zur Behandlung mit einem Molekül der Taxoid-Familie in Kombination mit einer Verbindung, die den TGF-β Signalweg inhibiert, oder zur Bestimmung ob ein mit einem Tumor befallener Patient dafür empfindlich ist, um von einer Behandlung mit einem Molekül der Taxoid-Familie in Kombination mit einer Verbindung, die den TGF-β Signalweg inhibiert, zu profitieren, wobei das Verfahren umfasst:
(a) Bestimmung des Expressionswerts von SHISA3 in einer Krebsprobe des Patienten,
(b) Vergleichen des Expressionswerts von SHISA3 mit einem Referenzexpressionswert, wobei die Unterexpression von SHISA3 vorhersagt, dass eine Behandlung mit einem Molekül der Taxoid-Familie in Kombination mit einer Verbindung, die den TGF-β Signalweg inhibiert, für den Patienten angezeigt ist, und
(c) Auswählen von Patienten mit einer Unterexpression von SHISA3 als geeignet für die Behandlung mit einem Molekül der Taxoid-Familie in Kombination mit einer Verbindung, die den TGF-β Signalweg inhibiert.

2. Eine Verbindung, die den TGF-β Signalweg inhibiert, zur Verwendung in Kombination mit einem Molekül der Taxoid-Familie in der Behandlung eines Krebs in dem SHISA3 unterexprimiert ist im Vergleich mit einem Referenzexpressionswert.

3. Das Verfahren nach Anspruch 1 oder die Verbindung nach Anspruch 2, wobei der Krebs gegen ein Molekül der Taxoid-Familie resistent ist.

4. Das Verfahren nach Anspruch 1 oder 3 oder die Verbindung nach Anspruch 2 oder 3, wobei der Expressionswert von SHISA3 durch Messen der Menge des SHISA3-Proteins oder der SHISA3-mRNA bestimmt wird.

5. Das Verfahren nach einem der Ansprüche 1, 3 oder 4 oder die Verbindung nach einem der Ansprüche 2 bis 4, wobei der Referenzexpressionswert der Expressionswert von SHISA3 in Zelllinien oder Tumoren ist, die empfindlich für die Behandlung mit dem Molekül der Taxoid-Familie sind.

6. Das Verfahren oder die Verbindung nach Anspruch 5, wobei die Zelllinie IGR-CaP1 ist.

7. Das Verfahren nach einem der Ansprüche 1 oder 3 bis 6 oder die Verbindung nach einem der Ansprüche 2 bis 6, wobei das Molekül der Taxoid-Familie ausgewählt ist aus der Gruppe bestehend aus Paclitaxel (Taxol®), Docetaxel (Taxotere®), Larotaxel, Cabazitaxel (XRP6258), BMS-184476, BMS-188797, BMS-275183, Ortataxel, RPR 109881A, RPR 116258, NBT-287, PG-Paclitaxel, ABRAXANE®, Tesetaxel, IDN 5390, Taxoürexin, GHA-Paclitaxel, und MAC-321, Genexol-PM, LEP (LEP-ETU), Tocosol® Paclitaxel, SB-T-1213, SB-T-1214, SB-T-1216, SB-T-1217, SB-T-121303021, SB-T-12130301, und einer Kombination davon.

8. Das Verfahren nach einem der Ansprüche 1 oder 3 bis 7 oder die Verbindung nach einem der Ansprüche 2 bis 7, wobei das Molekül der Taxoid-Familie Docetaxel ist.

9. Das Verfahren nach einem der Ansprüche 1 oder 3 bis 8 oder die Verbindung nach einem der Ansprüche 2 bis 8, wobei der Krebs ausgewählt ist aus der Gruppe bestehend aus Prostatakrebs, Lungenkrebs, Brustkrebs, Magenkrebs, Nierenkrebs, Eierstockkrebs, Leberzellkrebs, Osteosarkom, Melanom, Hypopharynxkrebs, Speiseröhrenkrebs, Gebärmutterschleimhautkrebs, Gebärmutterhalskrebs, Bauchspeicheldrüsenkrebs, Leberkrebs, Gehirntumor, neuroendokrine Tumore, bösartiger Tumor des Muskels, Nebennierenkrebs, Schilddrüsenkrebs, Gebärmutterkrebs, Hautkrebs, Blasenkrebs, und Kopf- und Halskrebs, vorzugsweise Prostatakrebs.

10. Das Verfahren nach einem der Ansprüche 1 oder 3 bis 9 oder die Verbindung nach einem der Ansprüche 2 bis 9, wobei die Verbindung, die den TGF-β Signalweg inhibiert, ein Inhibitor des TGF-β-Rezeptors, vorzugsweise ein Inhibitor des TGF-β I-Rezeptors ist.

11. Das Verfahren oder die Verbindung nach Anspruch 10, wobei der Inhibitor des TGF-β I-Rezeptors ausgewählt werden kann aus der Gruppe bestehend aus LY2157299 (Galunisertib), SB431542, LY2109761, SB525334, SB505124, RepSox, GW788388, LY364947, A 83-01, SD 208, D 4476, TGF-β RI Kinase Inhibitor VIII, Ki268994, vorzugsweise ist der Inhibitor des TGF-β I-Rezeptor LY2157299.

12. Verwendung eines Kits zur Auswahl eines mit einem Tumor befallenen Patienten zur Behandlung mit einem Molekül der Taxoid-Familie in Kombination mit einer Verbindung, die den TGF-β Signalweg inhibiert und/oder Durchmustern oder Identifizieren einer geeigneten Verbindung zur Verbesserung der Behandlung eines Krebs mit einem Molekül der Taxoid-Familie, wobei der Kit Nachweisvorrichtungen umfasst, ausgewählt aus der Gruppe bestehend aus einem Primerpaar, einer Sonde und einem für SHISA3 spezifischen Antikörper, und einer Kombination davon.

13. Verwendung des Expressionswerts von SHISA3 als Marker zur Auswahl eines mit einem Tumor befallenen Patienten zur Behandlung mit einem Molekül der Taxoid-Familie in Kombination mit einer Verbindung, die den TGF-β Signalweg inhibiert.

## Revendications

1. Méthode *in vitro* pour sélectionner un patient affecté par une tumeur pour un traitement avec une molécule de la famille des taxoïdes en combinaison avec un composé inhibant la voie de signalisation de TGF-β ou pour déterminer si un patient affecté par une tumeur est susceptible de tirer bénéfice d'un traitement avec une molécule de la famille des taxoïdes en combinaison avec un composé inhibant la voie de signalisation de TGF-β, laquelle méthode comprend :
(a) la détermination du niveau d'expression de SHISA3 dans un échantillon cancéreux provenant dudit patient,
(b) la comparaison du niveau d'expression de SHISA3 avec un niveau d'expression de référence, dans laquelle la sous-expression de SHISA3 est prédictive du fait qu'un traitement avec une molécule de la famille des taxoïdes en combinaison avec un composé inhibant la voie de signalisation de TGF-β est indiqué pour ledit patient, et
(c) la sélection de patients avec une sous-expression de SHISA3 comme convenant pour un traitement avec une molécule de la famille des taxoïdes en combinaison avec un composé inhibant la voie de signalisation de TGF-β.

2. Composé inhibant la voie de signalisation de TGF-β pour une utilisation en combinaison avec une molécule de la famille des taxoïdes dans le traitement d'un cancer dans lequel SHISA3 est sous-exprimée en comparaison avec un niveau d'expression de référence.

3. Méthode selon la revendication 1 ou composé selon la revendication 2, dans lequel ledit cancer est résistant à une molécule de la famille des taxoïdes.

4. Méthode selon la revendication 1 ou 3 ou composé selon la revendication 2 ou 3, dans lequel le niveau d'expression de SHISA3 est déterminé par mesure de la quantité de protéine SHISA3 ou d'ARNm de SHISA3.

5. Méthode selon l'une quelconque des revendications 1, 3 et 4 ou composé selon l'une quelconque des revendications 2 à 4, dans lequel le niveau d'expression de référence est le niveau d'expression de SHISA3 dans des lignées cellulaires ou des tumeurs sensibles à un traitement par la molécule de la famille des taxoïdes.

6. Méthode ou composé selon la revendication 5, dans lequel la lignée cellulaire est IGR-CaP1.

7. Méthode selon l'une quelconque des revendications 1 et 3 à 6 ou composé selon l'une quelconque des revendications 2 à 6, dans lequel la molécule de la famille des taxoïdes est choisie dans le groupe constitué par le paclitaxel (taxol®), le docétaxel (taxotère®), le larotaxel, le cabazitaxel (XRP6258), BMS-184476, BMS-188797, BMS-275183, l'ortataxel, RPR 109881A, RPR 116258, NBT-287, le PG-paclitaxel, l'ABRAXANE®, le Tésétaxel, IDN 5390, la Taxoprexine, le DHA-paclitaxel, et MAC-321, le Génexol-PM, LEP (LEP-ETU), le Tocosol® paclitaxel, SB-T-1213, SB-T-1214, SB-T-1216, SB-T-1217, SB-T-121303, SB-T-121303021, SB-T-12130301, et une combinaison de ceux-ci.

8. Méthode selon l'une quelconque des revendications 1 et 3 à 7 ou composé selon l'une quelconque des revendications 2 à 7, dans lequel la molécule de la famille des taxoïdes est le docétaxel.

9. Méthode selon l'une quelconque des revendications 1 et 3 à 8 ou composé selon l'une quelconque des revendications 2 à 8, dans lequel le cancer est choisi dans le groupe constitué par un cancer de la prostate, un cancer du poumon, un cancer du sein, un cancer gastrique, un cancer rénal, un cancer ovarien, un cancer hépatocellulaire, un ostéosarcome, un mélanome, un cancer de l'hypopharynx, un cancer de l'oesophage, un cancer de l'endomètre, un cancer du col de l'utérus, un cancer du pancréas, un cancer du foie, un cancer du cerveau, une tumeur neuroendocrinienne, une tumeur maligne musculaire, un cancer des glandes surrénales, un cancer de la thyroïde, un cancer de l'utérus, un cancer de la peau, un cancer de la vessie, et un cancer de la tête et du cou, de préférence un cancer de la prostate.

10. Méthode selon l'une quelconque des revendications 1 et 3 à 9 ou composé selon l'une quelconque des revendications 2 à 9, dans lequel le composé inhibant la voie de signalisation de TGF-β est un inhibiteur de récepteur de TGF-β, de préférence un inhibiteur du récepteur de TGF-β de type I.

11. Méthode ou composé selon la revendication 10, dans lequel l'inhibiteur du récepteur de TGF-β de type I peut être choisi dans le groupe constitué par : LY2157299 (Galunisertib), SB431542, LY2109761, SB525334, SB505124, RepSox, GW788388, LY364947, A 83-01, SD 208, D 4476, l'inhibiteur VIII de kinase de TGF-β RI, Ki268994, de préférence l'inhibiteur du récepteur de TGF-β de type I est LY2157299.

12. Utilisation d'un kit pour sélectionner un patient affecté par une tumeur pour un traitement avec une molécule de la famille des taxoïdes en combinaison avec un composé inhibant la voie de signalisation de TGF-β et/ou pour cribler ou identifier un composé convenant pour améliorer le traitement d'un cancer par une molécule de la famille des taxoïdes, dans laquelle le kit comprend des moyens de détection choisis dans le groupe constitué par une paire d'amorces, une sonde et un anticorps spécifique de SHISA3, et une combinaison de ceux-ci.

13. Utilisation du niveau d'expression de SHISA3 en tant que marqueur pour sélectionner un patient affecté par une tumeur pour un traitement avec une molécule de la famille des taxoïdes en combinaison avec un composé inhibant la voie de signalisation de TGF-β.
